# EUROPEAN PATENT SPECIFICATION

(11) **EP 4 499 875 B1**
(45) Date of publication and mention of the grant of the patent: **12.03.2025**
(21) Application number: 23729398.0
(22) Date of filing: 30.05.2023
(51) Int. Cl.: C12Q 1/6883, C12Q 1/6886

(54) **BIOMARKERS OF IL7R MODULATOR ACTIVITY**
BIOMARKER DER IL7R-MODULATORAKTIVITÄT
BIOMARQUEURS DE L'ACTIVITÉ DE MODULATEURS IL7R

(30) Priority: 30.05.2022 EP 22305790
(43) Date of publication of application: 05.02.2025
(73) Proprietor: OSE IMMUNOTHERAPEUTICS, 44200 Nantes (FR)
(72) Inventor: POIRIER, Nicolas, 44119 GRANDCHAMPS DES FONTAINES (FR); GIRAULT, Isabelle, 44000 NANTES (FR)
(74) Representative: Ernest Gutmann - Yves Plasseraud S.A.S.
(86) International application number: PCT/EP2023/064461
(87) International publication number: WO 2023/232826

(56) References cited:
- RIVIÈRE ELODIE ET AL: "Interleukin-7/Interferon Axis Drives T Cell and Salivary Gland Epithelial Cell Interactions in Sjögren's Syndrome", vol. 73, no. 4, 15 October 2020 (2020-10-15), US, pages 631 - 640, XP055979047, ISSN: 2326-5191, Retrieved from the Internet <URL:https://onlinelibrary.wiley.com/doi/full-xml/10.1002/art.41558> DOI: 10.1002/art.41558
- PRISCILA P ZENATTI ET AL: "Oncogenic IL7R gain-of-function mutations in childhood T-cell acute lymphoblastic leukemia", NATURE GENETICS, vol. 43, no. 10, 4 September 2011 (2011-09-04), New York, pages 932 - 939, XP055663629, ISSN: 1061-4036, DOI: 10.1038/ng.924
- JÄGER J ET AL: "IL7RA haplotype-associated alterations in cellular immune function and gene expression patterns in multiple sclerosis", GENES AND IMMUNITY, NATURE PUBLISHING GROUP, GB, vol. 14, no. 7, 29 August 2013 (2013-08-29), pages 453 - 461, XP037768626, ISSN: 1466-4879, [retrieved on 20130829], DOI: 10.1038/GENE.2013.40
- OLIVEIRA MARIANA L ET AL: "IL-7R-mediated signaling in T-cell acute lymphoblastic leukemia: An update", ADVANCES IN BIOLOGICAL REGULATION, ELSEVIER, AMSTERDAM, NL, vol. 71, 19 September 2018 (2018-09-19), pages 88 - 96, XP085582567, ISSN: 2212-4926, DOI: 10.1016/J.JBIOR.2018.09.012

## Description

### FIELD OF THE INVENTION

The present invention relates to a method of evaluating or predicting a therapeutic response to the treatment with an IL7R modulator such as IL7R antagonist or agonist in a patient, more particularly to the identification of biomarkers for evaluating or predicting whether an IL7R modulator would be effective for treating a patient. The present invention also relates to a method for screening a compound that would be effective for treating a patient.

### BACKGROUND OF THE INVENTION

IL-7 is the most important cytokine for the homeostasis, proliferation, differentiation, and survival of both naive and memory T cells. IL-7 signals through the IL-7 receptor (IL-7R), which is composed of an alpha chain (IL-7Rα), also called CD127, and a common cytokine receptor gamma chain (IL-2Rγ, CD132). IL-7R activation induces proliferative and anti-apoptotic signals mainly by activating the JAK-STAT pathway. Some studies have reported that IL-7 canal also activates the PI3K or MAPK/ERK pathways, suggesting that IL-7 could use different signaling pathways depending both on cellular type and the physiological status of the cell.

IL-7 and IL-7R are implicated in the pathogenesis of different disease states mediated by dysfunctions of lymphoid function, for instance autoimmune diseases such as diabetes and multiple sclerosis (Lee, L.F. et al. Proc Natl Acad Sci U S A 109, 12674-12679 (2012)), chronic inflammatory diseases such as rheumatoid arthritis, ankylosing spondylitis and inflammatory bowel disease (Churchman, S.M. & Ponchel, F. Rheumatology (Oxford) 47, 753-759 (2008); Krzystek-Korpacka, M. et al. Cancer Immunol Immunother 66, 171-179 (2017); Anderson, C.A. et al. Nat Genet 43, 246-252 (2011); Gracey, E. et al. Ann Rheum Dis 75, 2124-2132 (2016)) and hematological cancers (Oliveira, M.L., et al. Adv Biol Regul 71, 88-96 (2018)).

CD127 is fully expressed on naive and memory T cells but lowly expressed in FoxP3+ regulatory T cells (Tregs). This constitutes a unique opportunity to selectively target pathogenic effectors while preserving natural regulators (Liu W, et al. J. Exp. Med. 2006; 203:1701-1711; Seddiki N, et al. J. Exp. Med. 2006; 203:1693-1700; Michel L, et al. J. Clin. Invest. 2008;118:3411-3419). A number of antagonists of IL7R are currently being developed. Although several studies have been performed to analyze gene expression profile and identify biomarkers either *in vitro* or *in vivo* for predicting or monitoring therapeutic response such as anti-PD1 antibodies in cancer patients, gene signature relating to IL7R antagonist or agonist treatment are not known or not publicly available according to Applicant's knowledge. IL7 signaling pathway is complex and involves several genes. Indeed, 481 genes were differentially expressed in human PBMCs incubated with anti-human IL-7Rα mAbs compared to control conditions (Belarif L. et al. Nat Commun. 2018 Oct 26;9(1):4483).

Thus, there is a need to identify drug activity biomarkers to accurately monitor a patient's response to such drug in clinical trials. Such markers would facilitate the individualization of therapy for each patient.

Rivière et al. 2020, Arthritis & Rheumatology 73(4):631-640, discloses that treatment with an anti-IL-7R antibody lead to inhibition of IL-7 signaling as assessed by decreased expression of IFITM1 and MX1.

### SUMMARY OF THE INVENTION

A method for analyzing gene expression profile in a subject treated with anti-IL7R antagonist antibody identified 41 genes differentially expressed. On the other hand, a method for analyzing gene expression profile in human cells treated *in vitro* with IL7 cytokine identified 56 genes differentially expressed. Thus, it remains a need to identify a limited number of drug-related biomarkers to assess easily anti-IL7R antagonist or agonist efficacy.

Moreover, the applicant has previously shown that only a small percentage of IL7-induced transcriptional modification were common to mice and human therefore highlighting the poor translatability of murine studies to the human when investigating IL7-IL7R signaling pathway. Thus, *in vitro* gene signature on mouse models cannot be performed to identify the drug-related biomarkers for use in human.

In order to solve these problems, the inventors in the present application developed a new powerful method done in human peripheral blood mononuclear cells to identify highly relevant drug-related biomarkers (also referred herein gene signature) to monitor IL7R agonist or antagonist such as anti-IL7R antagonist antibody activity. They used a comparative screen that allows to identify a very limited number of genes that can be used as highly efficient drug-related biomarkers to monitor accurately the efficacy of IL7R antagonist or agonist treatment. By measuring the expression level of at least one of these 4-6 target genes, the efficiency of an IL7R antagonist or agonist compound can be assessed much more easily, and at a lower cost to avoid risk in clinical trials. The use of these biomarkers can be extended to assess the likelihood of therapeutic responses to said IL7R modulator. Thus, the selection of treatment compounds can be done much more selectively.

The invention is defined by the scope of the appended claims.

The present invention relates to an *in vitro* method for evaluating the therapeutic response of IL7R modulator such as IL7R antagonist (e.g. anti-IL7R antagonist antibody or antigen binding fragment thereof) or IL7R agonist (e.g. IL7) treatment in a human patient comprising the steps of determining the gene expression profile of each of the following genes: *BCL2, CISH, PTGER2* and *DPP4* genes, preferably further FLT3LG or SOCS2 genes, more preferably *BCL2, CISH, PTGER2, DPP4, FLT3LG* and *SOCS2* genes in a sample of patient having received at least one dose of IL7R antagonist (e.g. anti-IL7R antagonist antibody or antigen binding fragment thereof) or IL7R agonist (e.g. IL7), wherein a lower or higher gene expression profile of said genes in said patient sample as compared to a control value is indicative that the patient is likely responsive to the antagonist or agonist treatment respectively. This set of genes corresponds to a unique signature to monitor IL7R modulator activity.

In another aspect, the present invention relates to an IL7R antagonist (e.g. anti-IL7R antagonist antibody or antigen binding fragment thereof) for use in the treatment of disease associated with an increase of the IL7R signaling pathway induced by IL7 in a human patient in need thereof, wherein said antagonist is administered to said patient after the patient has been administered with at least one dose of said antagonist (e.g. anti-IL7R antagonist antibody or antigen binding fragment thereof) and when said patient is evaluated as likely responsive to said antagonist (e.g. anti-IL7R antagonist antibody or antigen binding fragment thereof) treatment in a method as described above, preferably wherein said disease associated with an increase of the IL7R signaling pathway induced by IL7 is selected from the group consisting of an autoimmune disease, an inflammatory disease, an allergic disease, a cancer disease, an infectious disease, a respiratory disease, and a disease related to transplantation, involving the activation or proliferation of CD127 positive diseased cells, in particular an autoimmune disease or an inflammatory disease, involving the activation or proliferation of CD127 positive cells, more preferably a chronic inflammatory disease such as Sjogren's syndrome or inflammatory bowel disease such as ulcerative colitis.

In another aspect, the present invention relates to an IL7R agonist for use in the treatment of disease associated with impaired healthy T-cell activity, preferably disorder(s) in which immune response stimulation is required, more preferably a cancer disease or an infectious disease which does not itself depend on the IL7R signaling pathway for its development in a human patient in need thereof, wherein said agonist is administered to said patient after the patient has been administered with at least one dose of said agonist and when said patient is evaluated as likely responsive to said agonist treatment in a method as described above.

The present invention also relates to an *in vitro* method for assessing a likelihood of a therapeutic response in a human patient to an IL7R modulator such as IL7 receptor antagonist (e.g., anti-IL7R antagonist antibody or antigen binding fragment thereof) or agonist treatment comprising the steps of:
a) culturing in presence of said IL7R modulator such as IL7R antagonist (e.g., anti-IL7R antagonist antibody or antigen binding fragment thereof) or IL7R agonist a sample comprising cells previously collected from a patient prior to said IL7R modulator treatment,
b) determining the gene expression profile of each of the following genes: *BCL2, CISH, PTGER2* and *DPP4* genes, preferably further FLT3LG or SOCS2 genes, more preferably *BCL2, CISH, PTGER2, DPP4, FLT3LG* and *SOCS2* genes in said cultured sample, wherein a lower or higher gene expression profile of said gene(s) in cultured sample as compared to a control value is indicative that the patient is likely responsive to the antagonist (e.g. anti-IL7R antagonist antibody or antigen binding fragment thereof) or agonist treatment respectively.

In another aspect, the present invention relates to an IL7R antagonist (e.g. anti-IL7R antagonist antibody or antigen binding fragment thereof) for use in the treatment of a disease associated with an increase of the IL7R signaling pathway induced by IL7 in a human patient in need thereof wherein said patient was previously assessed to be likely responsive to said antagonist (e.g. anti-IL7R antagonist antibody or antigen binding fragment thereof) treatment in a method as defined above, preferably wherein said disease associated with an increase of IL7R signaling pathway induced by IL7 is selected from the group consisting of: an autoimmune disease, an inflammatory disease, an allergic disease, a cancer disease, an infectious disease, a respiratory disease, and a disease related to transplantation, involving the activation or proliferation of CD127 positive diseased cells, in particular an autoimmune disease or an inflammatory disease, involving the activation or proliferation of CD127 positive cells, more preferably a chronic inflammatory disease such as Sjogren's syndrome or inflammatory bowel disease such as ulcerative colitis.

In another aspect, the present invention relates to an IL7R agonist for use in the treatment in a human patient of a disease associated with impaired healthy T-cell activity, preferably a cancer disease or an infectious disease which does not itself depend on the IL7R pathway for its development wherein said patient was previously assessed to be likely responsive to said IL7R agonist treatment in a method as defined above wherein said patient was previously assessed to be likely responsive to said agonist (e.g. anti-IL7R antagonist antibody or antigen binding fragment thereof) treatment in a method as defined above .

The present invention also relates to an *in vitro* method for selecting an IL7R modulator, such as IL7R antagonist or IL7R agonist compound effective in the treatment of a disease associated with an increase of IL7R signaling pathway induced by IL7 or a disease associated with impaired healthy T-cell activity comprising the steps of:
a) culturing human a cell in presence of said compound,
b) determining the gene expression profile of each of the following genes: *BCL2, CISH, PTGER2* and *DPP4* genes, preferably further FLT3LG or SOCS2 genes, more preferably *BCL2, CISH, PTGER2, DPP4, SOCS2* and *FLT3LG* genes in said cell, and
c) selecting an antagonist or agonist compound that induces a lower or higher gene expression profile of said gene(s) respectively in said cell as compared to a control value.

In another aspect the present invention relates to a kit consisting of a set of reagents that specifically detects the gene expression profile of each of the following genes: *BCL2, CISH, PTGER2* and *DPP4* genes, BCL2, CISH, PTGER2, DPP4 and FLT3LG genes, BCL2, CISH, PTGER2, DPP4 and SOCS2 genes or *BCL2, CISH, PTGER2, DPP4, SOCS2* and *FLT3LG* genes, preferably wherein said reagents are primer pairs and/or probes specific of each gene.

### DETAILED DESCRIPTION OF THE INVENTION

The term "biomarker" refers to a distinctive biological or biologically derived indicator (i.e., cellular, biochemical, molecular, genetic, protein, metabolite, specific post-translational modification or physiological or physical sign) of a process, event or condition. According to the present disclosure said biomarker may refer to a gene signature. A gene signature is a single or combined group of genes in a cell with a uniquely characteristic gene expression profile that occurs as a result of an altered or unaltered biological process or pathogenic medical condition.

The terms "subject" and "patient" are used interchangeably herein and refer to both human and non-human animals. As used herein, the term "patient" denotes a mammal, such as a rodent, a feline, a canine, and a primate. Preferably, a patient according to the invention is a human.

The term "patient sample" means any biological sample derived from a patient. Examples of such samples include tissue sample, cell samples, organs, biopsies, preferably tumor tissue containing infiltrated immune cells from a cancer patient or a blood patient sample. Preferred biological samples are cell sample, preferably blood cells sample such as human isolated peripheral blood mononuclear cells.

The term "likely responder", or "likely responsive to a treatment" refers to a subject in whom the onset of at least one of the symptoms of the condition to be treated is delayed or prevented, upon or after treatment, or whose symptoms or at least one of the symptoms stabilize, diminish or disappear.

"Therapeutic response" refers to the consequence of a medical treatment in a patient, the results of which are judged to be useful or favorable. For instance, a therapeutic response may be the delay or the prevention of at least one of the symptoms, upon or after treatment, or may be that the symptoms or at least one of the symptoms in patient stabilize, diminish or disappear.

According to the present disclosure, by " therapeutic response in patient treated with an IL7R modulator" or "IL7R modulator therapeutic response", it is meant that the performed steps of IL7R modulator such as an IL7R antagonist (e.g. anti-IL7R antagonist antibody or antigen binding fragment thereof) or IL7R agonist (e.g. IL7) administration result in improving the clinical condition of an animal or a human patient in need thereof, who suffers from disorder(s) associated with an increase of the IL7R signaling pathway induced by IL7 (also referred here IL7-IL7R pathway), i.e; signaling pathway induced by IL7-IL7 receptor interaction involving the activation and/or proliferation of CD127 positive cells or a disease associated with impaired healthy T-cell activity respectively. Such treatment aims at improving the clinical status of the animal or human patient, by eliminating or lowering the symptoms associated with the disorder(s) related to the IL-7 or TSLP (Thymic Stromal Lymphopoietin), in a preferred embodiment by eliminating or lowering the symptoms associated with the disorder(s) related to IL7R signaling pathway induced by IL7 or a disease associated with impaired healthy T-cell activity.

In a particular embodiment, the therapeutic response in patient treated with an IL7R antagonist (e.g. anti-IL7R antagonist antibody or antigen binding fragment thereof) refers to IL7R antagonist administration that results in improving clinical condition of a patient who suffers from disorders associated with an increase of the IL7R signaling pathway induced by IL-7 such as an autoimmune disease, an inflammatory disease, an allergic disease, a cancer disease, an infectious disease, a respiratory disease, and a disease related to transplantation, involving the activation or proliferation of CD127 positive diseased cells, in particular an autoimmune disease or an inflammatory disease, involving the activation or proliferation of CD127 positive cells, more preferably a chronic inflammatory disease such as Sjogren's syndrome or inflammatory bowel disease such as ulcerative colitis.

In another particular embodiment, the therapeutic response in patient treated with an IL7R agonist (e.g. IL7) refers to IL7R agonist administration that results in improving clinical condition of a patient who suffers from disorder(s) or disease(s) associated with impaired healthy T-cell activity, preferably disorder(s) in which immune response stimulation is required, more preferably a cancer disease or an infectious disease which does not itself depend on the IL7R pathway for its development.

Typically, a IL7R modulator may be a compound that has an IL7R antagonist or agonist properties for IL7-IL7 receptor interaction and modulates (increases or decreases) IL7/IL7R mediated signaling (the IL7R signaling pathway induced by IL7).

In a preferred embodiment, said IL7R modulator (e.g. IL7R antagonist or IL7R agonist) can be selected from the group consisting of: protein, polypeptide or peptide modulator such as components of the IL7-IL7R signaling pathway (e.g. IL7), variants or derivative thereof (fragments, fusion proteins, soluble proteins, dominant negative mutants), antibodies directed to component of IL7-IL7R signaling pathway including fragment and expressible derivative thereof and antigen-binding antibody mimetics.

Binding of IL-7 to IL-7R triggers the activation of several signaling pathways, including the Janus kinases (JAK) -1 and -3, signal transducer and activator of transcription 5 (STATS) and phosphatidylinostol 3-kinase (PI3-k). STAT1 and STAT3 pathways are reported to be activated, although they do not seem to be the main pathways. The activation of the STAT5 pathway is required for the induction of the anti-apoptotic protein Bcl-2 and the prevention of the entry of the pro-apoptotic protein Bax in the mitochondrion and thus for survival of thymic developing T cell precursors. The activation of the PI3-k pathway results in the phosphorylation and cytoplasmic retention of the pro-apoptotic protein Bad.

In a particular embodiment, IL7-R signaling pathway induced by IL7 can be identified by measuring STAT5 phosphorylation as described in the Examples of WO2018/104483. The IL7-induced phosphorylation of STAT5 is a marker of IL7-R activation. A compound antagonizing IL7-IL7-R interaction is expected to decrease IL7-induced phosphorylation of STAT5 and a compound agonizing IL7-IL7-R interaction is expected to increase IL7-induced phosphorylation of STATS.

In particular embodiments, the antagonist of IL7-R according to the present disclosure inhibits IL7-induced phosphorylation of STATS. In preferred embodiments, the inhibition of STAT5 phosphorylation is greater than 50 % at IL7R antagonist concentrations as low as 55 ng/ml and / or the inhibition of STAT5 phosphorylation is greater than 80 % at antibody concentrations as low as 100 ng/ml. Inhibition of STAT5 phosphorylation may be assessed by methods known to the skilled person and in particular by the method set forth in the Example 5, and/or in page 21, paragraphs [69] and [70] of WO2018/104483 and Example 3 of WO2015/189302. The agonist of IL7-R according to the present disclosure increases IL7-induced phosphorylation of STATS.

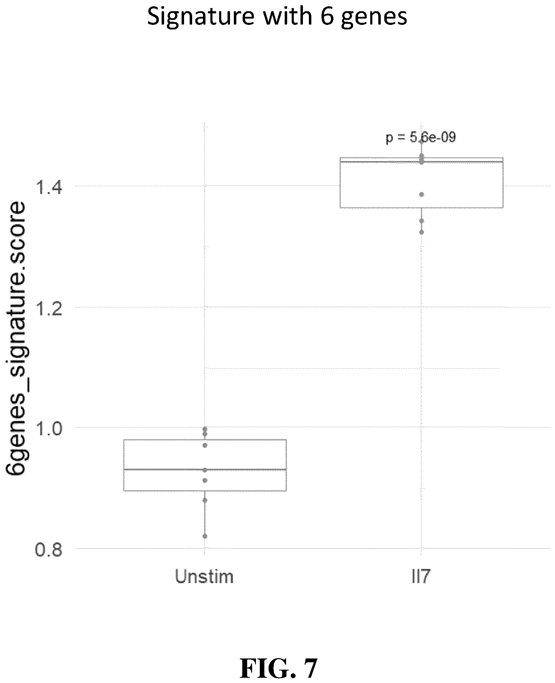

IL7R modulator such as IL7R antagonist (in particular antibody, antigen-binding fragment thereof) or agonist of the disclosure can be identified by measuring the activation of the PI3-K and/or ERK (Extracellular signal-regulated kinase) signaling pathways. In a particular embodiment, IL7R antagonist inhibits the activation and/or does not activate or increase the activation, of the PI3-k and/or ERK (Extracellular signal-regulated kinase) signaling pathways and in particular inhibits the phosphorylation and/or does not induce or increase the phosphorylation of PI3-k and/or ERK 1 and/or ERK 2. In particular, the IL7R antagonist provided herein does not induce the activation of the PI3-k and/or the ERK pathways (preferably of the PI3-k and the ERK pathway), and in particular does not induce the phosphorylation of PI3-k and/or of ERK 1 and/or ERK 2, more particularly does not induce the phosphorylation of PI3-k and of ERK 1 and of ERK 2. In particular, the IL7R antagonist, inhibits the activation of the PI3-k and/or the ERK pathways, and in particular inhibits the phosphorylation of PI3-k and/or of ERK 1 and/or ERK 2, more particularly inhibits the phosphorylation of PI3-k and of ERK 1 and of ERK 2. The activation of the pathways and/or phosphorylation of said proteins, may be tested by methods known to the skilled person and in particular by Western blotting as illustrated in Figure 7 and Example 8 of WO2018/104483, preferably by inhibition of the activation of PI3-k and/or ERK.

In another particular embodiment, IL7R agonist induces or increases the activation of the PI3-k and/or ERK (Extracellular signal-regulated kinase) signaling pathways and in particular induces or increases the phosphorylation of PI3-k and/or ERK 1 and/or ERK 2.

IL7R antagonist (in particular antibody, antigen-binding fragment thereof) or agonist of the disclosure can also be identified by measuring the internalization of CD127. In a particular embodiment, the IL7R antagonists provided herein inhibit the IL7-induced internalization of CD127 and IL7R agonists increase the IL7-induced internalization of CD127. The internalization of CD127 can be measured by determining cell surface expression level of CD127 in cells incubating with IL7R modulator (e.g. IL7R antagonist or agonist) in comparison to a control value such as cells not incubating with said modulator.

In a particular embodiement, when incubated with said IL7R antagonist, the presence of IL7 induces no decrease in the cell surface expression of CD127, or induces a less strong decrease in the cell surface expression of CD127 than cells incubated without antagonists. In particular embodiments, when incubated with said antagonists, the level of CD127 cell surface expression when cells are incubated at 37 °C for 15 minutes with 5 ng/mL IL7 is at least 80 %, preferably at least 90 % of the cell surface expression level in cells incubated without IL7. *In vitro,* the cell surface expression is preferably measured after a limited time as indicated above. Besides, as most cellular internalization processes are inhibited at low temperature, the effect is usually best observed at physiological temperature, in particular 37 °C. However, it is also contemplated to incubate cells at low temperature, in particular 4 °C.

In another preferred embodiment, the IL7R antagonist provided herein do not induce the internalization of CD127. Thus, the cell surface expression of CD127 in cells incubated in the presence of said IL7R antagonist is not reduced, or is not significantly reduced, relative to cell surface expression in cells incubated in otherwise identical conditions, but in the absence of the antagonist. In particular embodiments, when incubated at 37 °C for 30 to 45 minutes in the presence of 50 ng/mL of antagonist, the level of CD127 cell surface expression is at least 80 %, preferably at least 90 % of its level in cells incubated in the absence of the antagonist. This antagonist effect effect may be observed in the absence of IL7 (in both antibody-treated and -untreated cells), in the presence of IL7, and/or both.

The two CD127 internalization-related feature described above (i.e. inhibition of IL7-induced internalization or non-induction of internalization) may be further defined and/or tested as set forth in WO2015/189302 in particular in paragraphs [59]-[63] at pages 19-20 and in Figure 16 and Example 5.

For example, IL7R modulator according to the present disclosure can be identified by the following method comprising the steps consisting of:
a) providing a plurality of cells expressing CD127 on their surfaces,
b) incubating said cells with a candidate compound,
c) determining whether said candidate compound binds to and decrease or increase the biological activity of CD127 as described above (e.g., STAT5 phosphorylation, activation of the phosphatidylinositol 3-kinase and/or ERK signaling pathway, CD127 internalization), and
d) selecting the candidate compound that decreases or increases the biological activity of CD127 (e.g., STAT5 phosphorylation, activation of the phosphatidylinositol 3-kinase and/or ERK signaling pathway, CD127 internalization).

In a preferred embodiment, the IL7R modulator according to the present disclosure is an IL7R antagonist.

As used herein "IL7 receptor antagonist (e.g., anti-IL7R antagonist antibody or antigen-binding fragment thereof)" refers to compound (e.g. anti-IL7R antagonist antibody or antigen-binding fragment thereof) that has antagonist properties for IL7-IL7 receptor interaction, impairs IL7/IL7R mediated signaling cells. According to a particular embodiment, a IL7R antagonist according to the present disclosure further has antagonist properties toward interleukin 7 (IL7) thereby antagonizing access, i.e., binding of IL7 to CD127 on CD127 positive cells.

"Antagonist properties towards IL7-IL7-R interaction" means that IL7R antagonists (e.g., IL7R antagonist antibodies or antigen-binding fragments thereof), which target the IL7-Ralpha, have the effect of preventing the accessibility of the IL7 receptor expressed on CD127 cells, especially human effector T cells, in particular human memory T cells, for its binding partner IL7, especially human IL7. As a result of antagonizing binding of IL7, the IL7R antagonist leads to lymphopenia by preventing IL7-dependent thymic T cells generation. The antagonist properties may be in particular antagonism toward IL7-R signaling induced by IL7.

In a preferred embodiment, the IL7R antagonist provided herein does not bind CD127 in the TSLP (Thymic Stromal Lymphopoietin) receptor (i.e., does not bind CD127 when it is in a complex with the CRLF2, forming the TSLP receptor). Therefore, the antagonist provided herein does not interfere with TSLP-induced and / or TSLP receptor-mediated signaling. In another preferred embodiment, said IL7R antagonist is not capable of binding to non-T cells in whole blood; ((d) not capable of binding to CD127IL-7Ra expressed on non-T cells (e.g., monocytes) in whole blood, e.g., human whole blood".

In a particular embodiment, said IL7R antagonist may be an anti-IL7R antagonist antibody or antigen-binding fragment thereof, preferably a monoclonal antibody or antigen-binding fragment thereof which specifically bind against the alpha chain of the receptor of IL-7, preferably the alpha chain of the receptor of human IL-7. The alpha chain of the receptor for interleukin 7 (IL-7) is designated CD127 or p90 IL-7R, IL-7Ralpha or IL-7Rα (NCBI Accession Number: XP_005248356.1, updated on November 22, 2021). According to the present disclosure, said IL7R antagonist is preferably IL7Rα antagonist, more preferably anti-IL7Rα antagonist antibody or antigen-binding fragment thereof.

As used herein, the term "specifically binds to" or "binds specifically" refers to the ability of an antigen receptor to bind to an antigen with an affinity of at least about 1 × 10⁻⁶ M, 1 × 10⁻⁷ M,1 × 10⁻⁸ M, 1 × 10⁻⁹ M, 1 × 10⁻¹⁰ M, 1 × 10⁻¹¹ M, 1 × 10⁻¹² M, or more, and/or bind to a target with an affinity that is at least two-fold greater than its affinity for a nonspecific antigen. The affinity can be determined by various methods well known from one skilled in the art. These methods include, but are not limited to, Biacore Analysis, Blitz analysis and Scatchard plot.

In an embodiment, the antibody or antigen-binding fragment thereof according to the invention has a KD value inferior or equal to 10⁻⁸ M, preferably inferior or equal to 10⁻⁹ M for CD127, in particular for human CD127, more preferably inferior or equal to 1.10⁻¹⁰ M, as may be determined by biosensor analysis, particularly by Biacore Analysis.

The term "antibody" as used herein refers to immunoglobulin molecules and immunologically active portions of immunoglobulin molecules, i.e., molecules that contain an antigen binding site that immunospecifically binds an antigen. As such, the term antibody encompasses not only whole antibody molecules, but also antibody fragments as well as variants (including derivatives) of antibodies.

In natural antibodies of rodents and primates, two heavy chains are linked to each other by disulfide bonds, and each heavy chain is linked to a light chain by a disulfide bond. There are two types of light chains, lambda (λ) and kappa (κ). There are five main heavy chain classes (or isotypes) which determine the functional activity of an antibody molecule: IgM, IgD, IgG, IgA and IgE. Each chain contains distinct sequence domains. In typical IgG antibodies, the light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR).

The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FR) can participate in the antibody binding site or influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L- CDR3 and H-CDR1, H-CDR2, H-CDR3, respectively. An antigen-binding site, therefore, typically includes six CDRs, comprising the CDRs set from each of a heavy and a light chain V region. Framework Regions (FRs) refer to amino acid sequences interposed between CDRs. Accordingly, the variable regions of the light and heavy chains typically comprise 4 framework regions and 3 CDRs of the following sequence: FR1-CDR1-FR2-CDR2-FR3-CDR3-FR4.

The residues in antibody variable domains are conventionally numbered according to a system devised by Kabat et al. This system is set forth in Kabat et al., 1987, in Sequences of Proteins of Immunological Interest, US Department of Health and Human Services, NIH, USA (Kabat et al., 1992, hereafter "Kabat et al."). This numbering system is used in the present specification. The Kabat residue designations do not always correspond directly with the linear numbering of the amino acid residues in SEQ ID sequences. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence.

The term "monoclonal antibody" as used herein refers to a preparation of antibody molecules of single specificity. A monoclonal antibody displays a single binding specificity and affinity for a particular epitope. Accordingly, the term "human monoclonal antibody" refers to an antibody displaying a single binding specificity which has variable and constant regions derived from or based on human germline immunoglobulin sequences or derived from completely synthetic sequences. The method of preparing the monoclonal antibody is not relevant for the binding specificity.

The term "antigen-binding fragment" of an antibody (or simply "antibody fragment"), as used herein, refers to one or more fragments of an antibody that retain the ability to specifically bind to an antigen (e.g., IL7R) and in particular has antagonist properties for IL7-IL7 receptor interaction, impairs IL7/IL7R mediated signaling such as PI3K/Akt/mTOR and JAK/STAT pathways and may present cytotoxic activity against CD127 positive cells. It has been shown that the antigen-binding function of an antibody can be performed by fragments of a full-length antibody.

Examples of binding fragments encompassed within the term "antigen-binding fragment" of an antibody include a Fab fragment, a monovalent fragment consisting of the VL, VH, CL and CH1 domains; a F(ab)2 fragment, a bivalent fragment comprising two Fab fragments linked by a disulfide bridge at the hinge region; a Fd fragment consisting of the VH and CH1 domains; a Fv fragment consisting of the VL and VH domains of a single arm of an antibody; a dAb fragment (Ward et al., 1989 Nature 341:544-546), which consists of a VH domain, or any fusion proteins comprising such antigen-binding fragments. Furthermore, although the two domains of the Fv fragment, VL and VH, are coded for by separate genes, they can be joined, using recombinant methods, by a synthetic linker that enables them to be made as a single chain protein in which the VL and VH regions pair to form monovalent molecules (known as single chain Fv (scFv); see e.g., Bird et al., 1988 Science 242:423-426; and Huston et al., 1988 Proc. Natl. Acad. Sci. 85:5879-5883). Such single chain antibodies are also intended to be encompassed within the term "antigen-binding fragment" of an antibody. These antibody fragments are obtained using conventional techniques known to those of skill in the art, and the fragments are screened for utility in the same manner as are intact antibodies.

In a particular embodiment, said anti-IL7R antagonist antibody or antigen binding fragment thereof according to the present disclosure is N13B2h1, N13B2h2 or N13B2h3 antibody or antigen binding fragment thereof as described in WO2015/189302 or N13B2-hVL3, N13B2-hVL4, N13B2-hVL5 or N13B2-hVL6 antibody or antigen binding fragment thereof as described in WO2018/104483 and comprises: (a) a variable heavy chain comprising three CDRs wherein VHCDR1 comprises or consists of the amino acid sequence of SEQ ID NO: 1, VHCDR2 comprises or consists of the amino acid sequence of SEQ ID NO: 2, and VHCDR3 comprises or consists of the amino acid sequence of SEQ ID NO: 3 or 11 and; (b) a variable light chain comprising three CDRs wherein VLCDR1 comprises or consists of the amino acid sequence of SEQ ID NO: 4 or 12, VLCDR2 comprises or consists of the amino acid sequence of SEQ ID NO: 5 or 13 and VLCDR3 comprises or consists of the amino acid sequence of SEQ ID NO: 6

In a more particular embodiment, said anti-IL7R antagonist antibody or antigen binding fragment thereof according to the present disclosure is N13B2h3 antibody or antigen binding fragment thereof as described in WO2015/189302 or N13B2-hVL3, N13B2-hVL4, N13B2-hVL5 or N13B2-hVL6 antibody or antigen binding fragment thereof as described in WO2018/104483, preferably N13B2-hVL6 antibody or antigen binding fragment thereof as described in WO2018/104483 and comprises: (a) a variable heavy chain comprising three CDRs wherein VHCDR1 comprises or consists of the amino acid sequence of SEQ ID NO: 1, VHCDR2 comprises or consists of the amino acid sequence of SEQ ID NO: 2, and VHCDR3 comprises or consists of the amino acid sequence of SEQ ID NO: 3 and; (b) a variable light chain comprising three CDRs wherein VLCDR1 comprises or consists of the amino acid sequence of SEQ ID NO: 4, VLCDR2 comprises or consists of the amino acid sequence of SEQ ID NO: 5 and VLCDR3 comprises or consists of the amino acid sequence of SEQ ID NO: 6.

It is further contemplated that antibodies or antigen-binding fragment thereof may be further screened or optimized for their affinity binding and/or antagonist activities. In particular, it is contemplated that antibodies or antigen-binding fragment thereof may have 1, 2, 3, 4, 5, 6, or more alterations in the amino acid sequence of 1, 2, 3, 4, 5, or 6 CDRs of monoclonal antibodies provided herein. It is contemplated that the amino acid in position 1, 2, 3, 4, 5, 6, 7, 8, 9, or 10 of CDR1, CDR2, CDR3, CDR4, CDR5, or CDR6 of the VJ or VDJ region of the light or heavy variable region of antibodies may have an insertion, deletion, or substitution with a conserved or non-conserved amino acid. Such amino acids that can either be substituted or constitute the substitution are disclosed above.

In some embodiments, the amino acid differences are conservative substitutions, i.e., substitutions of one amino acid with another having similar chemical or physical properties (size, charge or polarity), which substitution generally does not adversely affect the biochemical, biophysical and/or biological properties of the antibody. In particular, the substitution does not disrupt the interaction of the antibody with the IL7R antigen and antagonistic property. Said conservative substitution(s) are advantageously chosen within one of the following five groups: Group 1-small aliphatic, non-polar or slightly polar residues (A, S, T, P, G); Group 2-polar, negatively charged residues and their amides (D, N, E, Q); Group 3-polar, positively charged residues (H, R, K); Group 4-large aliphatic, nonpolar residues (M, L, I, V, C); and Group 5-large, aromatic residues (F, Y, W).

In a particular embodiment, said anti-IL7R antagonist is N13B2h1, N13B2h2 or N13B2h3 antibody or antigen binding fragment thereof as described in WO2015/189302 and comprises a heavy chain variable domain comprising or consisting of the amino acid sequence selected from the group consisting of: SEQ ID NO: 14-17, and a light chain variable domain comprising or consisting of the amino acid sequence selected from the group consisting of SEQ ID NO: 18-21. Preferably, said anti-IL7R antagonist is N13B2h3 antibody or antigen binding fragment thereof as described in WO2015/189302 and comprises a heavy chain variable domain comprising or consisting of the amino acid sequence of: SEQ ID NO: 17, and a light chain variable domain comprising or consisting of the amino acid sequence of SEQ ID NO: 21 more preferably said antibody or antigen binding fragment thereof comprises a full heavy chain comprising or consisting of SEQ ID NO: 22 and a full light chain comprising or consisting of SEQ ID NO: 23.

In another particular embodiment, said anti-IL7R antagonist is N13B2-hVL3, N13B2-hVL4, N13B2-hVL5 or N13B2-hVL6 antibody or antigen binding fragment thereof as described in WO2018/104483 and comprises a heavy chain variable domain comprising or consisting of the amino acid sequence SEQ ID NO: 7, and a light chain variable domain comprising or consisting of the amino acid sequence selected from the group consisting of SEQ ID NO: 8, 24-26.

In a more preferred embodiment, said anti-IL7R antagonist antibody or antigen binding fragment thereof is N13B2-hVL6 antibody or antigen binding fragment thereof as described in WO2018/104483 and comprises a heavy chain variable domain comprising or consisting of the amino acid sequence SEQ ID NO: 7 and a light chain variable domain comprising or consisting of the amino acid sequence SEQ ID NO: 8, preferably said antibody or antigen binding fragment thereof comprises a full heavy chain comprising or consisting of SEQ ID NO: 9 and a full light chain comprising or consisting of SEQ ID NO: 10.

Anti-IL7R antagonist antibodies or antigen-binding fragments thereof with amino acid sequences having at least 90%, for example, at least 95%, 96%, 97%, 98%, or 99% identity to any one of the above defined amino acid sequences are also part of the present disclosure, typically anti- IL7R antagonist antibodies or antigen-binding fragments thereof have at least equal or higher antagonist activities for IL7-IL7R interaction as described above than said anti-IL7R antagonist antibodies or antigen-binding fragments thereof consisting of VH and VL, in particular of heavy chain and light chain of N13B2-hVL6 antibodies.

As used herein, the percent identity between the two sequences is a function of the number of identical positions shared by the sequences (i. e., % identity = number of identical positions/total number of positions x 100), taking into account the number of gaps, and the length of each gap, which need to be introduced for optimal alignment of the two sequences. The comparison of sequences and determination of percent identity between two sequences can be accomplished using a mathematical algorithm, as described below.

The percent identity between two amino acid sequences or nucleotide sequences can be determined using the algorithm of E. Meyers and W. Miller (Comput. Appl. Biosci., 4:11-17, 1988) which has been incorporated into the ALIGN program (version 2.0), using a PAM120 weight residue table, a gap length penalty of 12 and a gap penalty of 4. Alternatively, the percent identity between two amino acid sequences or nucleotide sequences can be determined using the Needleman and Wunsch (J. Mol, Biol. 48:444-453, 1970) algorithm which has been incorporated into the GAP program in the GCG software package (available at http://www.gcg.com), using either a Blossom 62 matrix or a PAM250 matrix, and a gap weight of 16, 14, 12, 10, 8, 6, or 4 and a length weight of 1, 2, 3, 4, 5, or 6. The percent identity between two nucleotide or amino acid sequences may also be determined using for example algorithms such as the BLASTN program for nucleic acid or amino acid sequences using as defaults a word length (W) of 11, an expectation (E) of 10, M=5, N=4, and a comparison of both strands.

In another particular embodiment of the disclosure, the IL7R antagonist (e.g., IL7R antagonist antibody or antigen-binding fragment thereof) have furthermore the property of being cytotoxic against human cells, especially human T cells expressing said receptor.

According to a particular embodiment of the disclosure, the cytotoxic IL7R antagonists are antibodies that has furthermore the capability to increase the Antibody Dependent Cellular Phagocytosis (ADCP) activity of CD127-positive tumor cells by macrophages, and does not have Antibody Dependent Cellular Cytotoxic (ADCC) activity, in particular on immune cells. In a particular embodiment of the invention, the anti-IL-7R antagonist antibody or antigen-binding fragment thereof, in particular anti-CD127 antibodies or fragment thereof to be used in the method of the invention, or for use according to the invention, has the capability to increase the Antibody Dependent Cellular Phagocytosis (ADCP) activity of CD127-positive tumor cells by macrophages, and preferably does not have Antibody Dependent Cellular Cytotoxic (ADCC) activity, in particular on immune cells.

In one another embodiment, the IL7R modulator (i.e., IL7R antagonist or IL7R agonist) is an antigen-binding antibody mimetic. As used herein the term "antigen-binding antibody mimetic" refers to artificial proteins, peptides and any chemical compounds with the capacity to bind antigens mimicking that of antibodies. Such mimetics comprise affitins and anticalins as well as aptamers (peptide aptamers and oligonucleotide aptamers).

In one embodiment, the IL7R modulator (i.e. antagonist or IL7R agonist) is an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library. The random sequence library is obtainable by combinatorial chemical synthesis of DNA. In this library, each member is a linear oligomer, eventually chemically modified, of a unique sequence. Peptide aptamers consists of a conformationally constrained antibody variable region displayed by a platform protein, such as E. coli Thioredoxin A that are selected from combinatorial libraries by two hybrid methods.

In some embodiments, the IL7R modulator (i.e., IL7R antagonist or IL7R agonist) is a polypeptide. The term "polypeptide" means herein a polymer of amino acids having no specific length. Thus, peptides, oligopeptides and proteins are included in the definition of "polypeptide" and these terms are used interchangeably throughout the specification, as well as in the claims. The term "polypeptide" does not exclude post-translational modifications that include but are not limited to phosphorylation, acetylation, glycosylation and the like.

In one embodiment, the polypeptide has a length comprised between 2 and 200 amino acids. In one embodiment, the polypeptide has a length comprised between 2 and 190, in particular between 10 and 180, between 10 and 170, between 10 and 160, between 10 and 150, between 10 and 140, between 10 and 130, between 10 and 120, between 10 and 110 amino acids. In one embodiment, the polypeptide has a length comprised between 10 and 100 amino acids. In one embodiment, the polypeptide has a length comprised between 50 and 100 amino acids. In one embodiment, the polypeptide has a length of 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100 amino acids.

In a particular embodiment, the polypeptide is a functional equivalent of IL7R. As used herein, a "functional equivalent" of IL7R is a compound which is capable of binding to at least one IL7 ligand, thereby preventing its interaction with IL7R. The term "functional equivalent" includes fragments, mutants, and muteins of IL7R. The term "functionally equivalent" thus includes any equivalent of IL7R obtained by altering the amino acid sequence, for example by one or more amino acid deletions, substitutions or additions such that the protein analogue retains the ability to bind to its ligand. Amino acid substitutions may be made, for example, by point mutation of the DNA encoding the amino acid sequence.

Functional equivalents include but are not limited to molecules that bind to a ligand of IL7R and comprise all or a portion of the extracellular domains of IL7R so as to form a soluble receptor that is capable to trap the ligand of IL7R. Thus, the functional equivalents include soluble forms of IL7R. A suitable soluble form of these proteins, or functional equivalents thereof, might comprise, for example, a truncated form of the protein from which the transmembrane domain has been removed by chemical, proteolytic or recombinant methods. Particularly, the functional equivalent consisting of a sequence having at least 80% identity, more particularly at least 85%, at least 86%, at least 87%, at least 88%, at least 89%, at least 90%, at least 91%, at least 92%, at least 93%, at least 94%, at least 95%, at least 96%, at least 97%, at least 98% and even more particularly at least 99% of identity with the corresponding protein over the entire length of the corresponding protein. As used herein, the term "corresponding protein" refers to the protein for which the functional equivalent has similar function. The percentages of identity to which reference is made in the presentation of the present invention are determined on the basis of a global alignment of sequences to be compared, that is to say, on an alignment of sequences over their entire length, using for example the algorithm of Needleman and Wunsch 1970. This sequence comparison can be done for example using the needle software by using the parameter "Gap open" equal to 10.0, the parameter "Gap Extend" equal to 0.5, and a matrix "BLOSUM 62". Software such as needle is available on the website ebi.ac.uk worldwide, under the name "needle". The term "a functionally equivalent fragment" as used herein also may mean any fragment or assembly of fragments of IL7R that binds to IL7. Accordingly, the present disclosure provides a polypeptide, in particular a functional equivalent, capable of inhibiting binding of IL7R to IL7, which polypeptide comprises consecutive amino acids having a sequence which corresponds to the sequence of at least a portion of an extracellular domain of IL7R, which portion binds to IL7. In some embodiments, the polypeptide, in particular the functional equivalent, corresponds to an extracellular domain of IL7R.

In some embodiments, the functional equivalent of IL7R is fused to a heterologous polypeptide to form a fusion protein. As used herein, a "fusion protein" comprises all or part (typically biologically active) of a functional equivalent of the present disclosure operably linked to a heterologous polypeptide (i.e., a polypeptide other than the same polypeptide). Within the fusion protein, the term "operably linked" is intended to indicate that the functional equivalent of the present disclosure and the heterologous polypeptide are fused in-frame to each other. The heterologous polypeptide can be fused to the N-terminus or C-terminus of the functional equivalent of the present disclosure.

In some embodiments, the functional equivalent of IL7R is fused to an immunoglobulin constant domain (Fc region) to form an immunoadhesin. Immunoadhesins can possess many of the valuable chemical and biological properties of human antibodies. Since immunoadhesins can be constructed from a human protein sequence with a desired specificity linked to an appropriate human immunoglobulin hinge and constant domain (Fc) sequence, the binding specificity of interest can be achieved using entirely human components. Such immunoadhesins are minimally immunogenic to the patient, and are safe for chronic or repeated use. In some embodiments, the Fc region is a native sequence Fc region. In some embodiments, the Fc region is a variant Fc region. In still another embodiment, the Fc region is a functional Fc region. As used herein, the term "Fc region" is used to define a C-terminal region of an immunoglobulin heavy chain, including native sequence Fc regions and variant Fc regions. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The adhesion portion and the immunoglobulin sequence portion of the immunoadhesin may be linked by a minimal linker. The immunoglobulin sequence typically, but not necessarily, is an immunoglobulin constant domain. The immunoglobulin moiety in the chimeras may be obtained from IgG1, IgG2, IgG3 or IgG4 subtypes, IgA, IgE, IgD or IgM, but typically IgG1 or IgG4. In some embodiments, the functional equivalent of IL7R and the immunoglobulin sequence portion of the immunoadhesin are linked by a minimal linker. As used herein, the term "linker" refers to a sequence of at least one amino acid that links the polypeptide and the immunoglobulin sequence portion. Such a linker may be useful to prevent steric hindrances. In some embodiments, the linker has 4; 5; 6; 7; 8; 9; 10; 11; 12; 13; 14; 15; 16; 17; 18; 19; 20; 21; 22; 23; 24; 25; 26; 27; 28; 29; 30 amino acid residues. However, the upper limit is not critical but is chosen for reasons of convenience regarding e.g. biopharmaceutical production of such polypeptides. The linker sequence may be a naturally occurring sequence or a non-naturally occurring sequence. If used for therapeutical purposes, the linker is typically non-immunogenic in the subject to which the immunoadhesin is administered. One useful group of linker sequences are linkers derived from the hinge region of heavy chain antibodies as described in WO 96/34103 and WO 94/04678. Other examples are poly-alanine linker sequences.

According to the present disclosure, a patient is likely responsive to an IL7R antagonist (e.g. anti-IL7R antagonist antibody or antigen-binding fragment thereof) treatment when said antagonist (e.g. anti-IL7R antagonist antibody or antigen-binding fragment thereof) has an IL7R antagonist effect in said patient that can be correlated with therapeutic response to said antagonist treatment.

In another particular embodiment, according to the present disclosure, a patient is likely responsive to an IL7R agonist (e.g., IL7) treatment when said has an IL7R agonist effect in said patient that can be correlated with therapeutic response to said agonist treatment.

According to the different method of the present disclosure, the analysis of gene expression profile of at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in patient sample previously treated *in vitro* or *in vivo* with IL7R modulator such as IL7R antagonist (e.g. anti-IL7R antagonist antibody or antigen-binding fragment thereof) or IL7R agonist can be used as biomarkers to determine the efficacy of IL7R modulator such as IL7R antagonist (e.g. anti-IL7R antagonist antibody or antigen-binding fragment thereof) or IL7R agonist in a patient and evaluate the therapeutic response or assess the likelihood of the therapeutic response of said patient to said antagonist (e.g. anti-IL7R antagonist antibody or antigen-binding fragment thereof) or agonist treatment or select an IL7R antagonist or agonist compound likely to be effective in said treatment.

The different methods according to the present disclosure thus involve determining the expression level of at least 1, 2, 3 or 4 genes selected from the group consisting of: *BCL2, CISH, PTGER2* and *DPP4* genes, preferably at least 1, 2, 3, 4, 5 and 6 genes selected from the group consisting of: *BCL2, CISH, PTGER2, DPP4, SOCS2* and *FLT3LG* genes in patient sample.

The term " determining the expression profile of at least 1, 2, 3 or 4 genes selected from the group consisting of: *BCL2, CISH, PTGER2* and *DPP4* genes " means that the expression level of at least 1, 2, 3 or 4 of said genes is assessed. In a particular embodiment, the expression level of each of all said genes is assessed.

Human *BCL2* gene, also named *BCL2 apoptosis regulator* (Gene ID: 596, updated on 20 June, 2021) encodes an integral outer mitochondrial membrane protein that blocks the apoptotic death of some cells such as lymphocytes. Human *BCL2* gene encodes two transcripts produced by alternative splicing BCL2 transcript variant alpha of 6881 bp (NCBI Reference Sequence: NM_000633.3, updated on 01 July, 2021) and BCL2 transcript variant beta of 1595 bp (NCBI Reference Sequence: NM_000657.3, updated on 01 July, 2021) encoding BCL2 isoform alpha protein (NCBI reference sequence: NP_000624.2, updated on 01 July, 2021) and BCL2 isoform beta protein (NCBI reference sequence: NP_000648.2, updated on 01 July, 2021) respectively.

Human *CISH* gene *(cytokine inducible SH2 containing protein,* Gene ID: 1154, updated on 20 June, 2021) encodes a protein containing SH2 domain and a SOCS box domain which belongs to cytokine-induced STAT inhibitor (CIS), also known as suppressor of cytokine signaling (SOCS) or STAT-induced STAT inhibitor (SSI) protein family. Human *CISH* gene encodes two transcripts, CISH transcript variant 1 of 2176 bp (NCBI Reference Sequence: NM_013324.7, updated on 01 July, 2021) and CISH transcript variant 2 of 2019 bp (NCBI Reference Sequence: NM_145071.4, updated on 01 July, 2021) encoding CISH isoform 1 protein (NCBI reference sequence: NP_037456.5, updated on 01 July, 2021) and CISH isoform 2 protein (NCBI reference sequence: NP_659508.1, updated on 01 July, 2021) respectively.

Human *PTGER2* gene (*Prostaglandin E receptor 2,* Gene ID: 5732 updated on 20 June, 2021) encodes a receptor for prostaglandin E2, a metabolite of arachidonic acid which has different biologic activities in a wide range of tissues. Human *PTGER2* gene encodes a transcript of 2548 bp (NCBI Reference Sequence: NM_000956.4, updated on 24 June 2021) encoding PTGER2 protein (NCBI Reference Sequence: NP_000947.2, updated on 24 June, 2021).

Human *DPP4* gene (*dipeptidyl peptidase 4,* Gene ID: 1803, updated on 28 June, 2021), also known as: *ADABP, ADCP2, CD26, DPPIV* and *TP103* encodes dipeptidyl peptidase 4, which is identical to adenosine deaminase complexing protein-2 and to the T-cell activation antigen CD26. Human *DPP4* gene encodes 4 transcripts: DPP4 transcript variant 1 of 3573 bp (NCBI Reference Sequence: NM_001935.4, updated on 29 June, 2021), DPP4 transcript variant 2 of 3570 bp (NCBI Reference Sequence: NM_ 001379604.1, updated on 1 July, 2021), DPP4 transcript variant 3 of 3567 bp (NCBI Reference Sequence: NM_001379605.1, updated on 30 June, 2021), DPP4 transcript variant 4 of 3519 bp (NCBI Reference Sequence: NM_001379606.1, updated on 01 July, 2021), which encodes 4 protein isoforms: DPP4 isoform 1 (NCBI Reference Sequence: NP_001926.2, updated on 29 June, 2021), DPP4 isoform 2 (NCBI Reference Sequence: NP_001366533.1, updated on 01 July, 2021), DPP4 isoform 3 (NCBI Reference Sequence: NP_001366534.1, updated on 30 June, 2021) and DPP4 isoform 4 (NCBI Reference Sequence: NP_001366535.1, updated on 01 July, 2021) respectively.

Human *SOCS2* gene (Gene ID: 8835, updated on 11 June 2021), also known as: CIS2, Cish2, SOCS-2, SSI-2, SSI2, STATI2 encodes a member of the suppressor of cytokine signaling (SOCS) family. Human *SOCS2* gene encodes five transcripts: SOCS2 transcript variant 1 of 2606 bp (NCBI Reference Sequence: NM_003877.5, updated on 27 June, 2021), SOCS2 transcript variant 2 of 2653 bp (NCBI Reference Sequence: NM_001270467.2, updated on 27 June, 2021), SOCS2 transcript variant 3 of 2581 bp (NCBI Reference Sequence: NM_001270468.2, updated on 27 June, 2021), SOCS2 transcript variant 4 of 2431 bp (NCBI Reference Sequence: NM_001270469.2, updated on 27 June, 2021), SOCS2 transcript variant 5 of 2888 bp (NCBI Reference Sequence: NM_001270470.1, updated on 23 June, 2021), SOCS2 transcript variant 6 of 2386 bp (NCBI Reference Sequence: NM_001270471.2, updated on 26 June, 2021). These transcripts encode SOCS2 protein of 198 amino acids (NCBI Reference Sequence: NP_003868.1, updated on 27 June 2021).

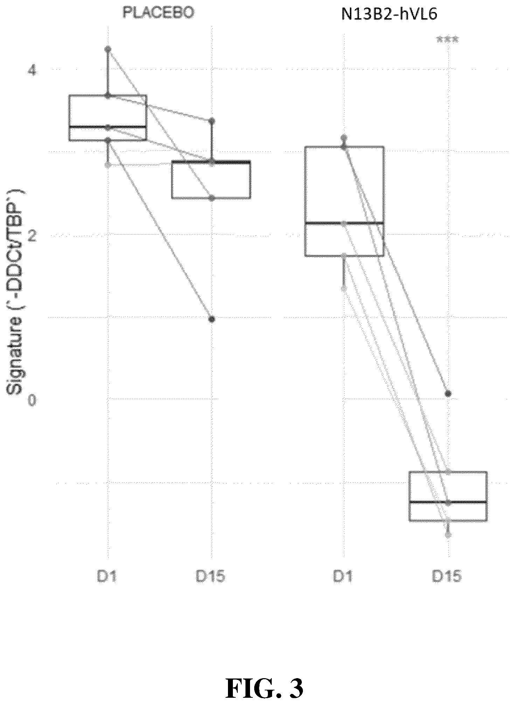

Human *FLT3LG* gene (*fms related receptor tyrosine kinase 3 ligand,* Gene ID: 2323 updated on 11 June 2021), also known as: FL, FLG3L, FLT3L encodes a protein which controls the development of dendritic cells. Human FLT3LG gene encodes fives transcripts: FLT3LG transcript variant 1 of 1101 bp (NCBI Reference Sequence: NM_001204502.2, updated on 27 June, 2021), FLT3LG transcript variant 2 of 1051 bp (NCBI Reference Sequence: NM_001204503.2, updated on 27 June, 2021), FLT3LG transcript variant 3 of 1050 bp (NCBI Reference Sequence: NM_001459.4, updated on 26 June, 2021), FLT3LG transcript variant 4 of 1078 bp (NCBI Reference Sequence: NM_001278637.2, updated on 03 May, 2021), FLT3LG transcript variant 5 of 1067 bp (NCBI Reference Sequence: NM_001278638.2, updated on 03 May, 2021). These transcripts encode two protein isoforms, FLT3LG isoform 1 precursor protein (NCBI Reference Sequence: NP_001450.2, updated on 26 June 2021) and FLT3LG isoform 2 (NCBI Reference Sequence: NP_001265566.1, updated on 3 May, 2021).

The gene expression profile of said genes may be determined by any suitable methods known by the person skilled in the art.

The term "gene expression profile" or "gene signature" refers to a pattern of expression of at least one gene of the set of genes as described above in a cell that occurs as a result of a biological process, herein the IL7R antagonist or agonist effect in a cell. According to the present disclosure, the gene expression profile of said biomarkers can be determined by measuring the relative amount of product genes expressed in cells treated with an ILR7 antagonist (e.g. anti-IL7R antagonist antibody or antigen-binding fragment thereof) or an IL7R agonist as described above and preferably by performing a single sample GSEA (ssGSEA) in GSVA algorithm, by calculating a gene set enrichment score per sample as the normalized difference in empirical cumulative distribution functions of gene expression ranks inside and outside the gene set.

Usually, these methods comprise measuring the quantity of mRNA or protein as described above. Methods for determining the quantity of mRNA are well known in the art. For example, the mRNA contained in the sample is first extracted according to standard methods, for example using lytic enzymes or chemical solutions or extracted by nucleic-acid-binding resins following the manufacturer's instructions. The extracted mRNA is then detected by hybridization (e.g., Northern blot analysis) and/or amplification (e.g., RT-PCR) by using primer pairs and probes specific to said genes as described in the examples of the present disclosure. Quantitative or semi-quantitative RT-PCR is preferred. In another particular embodiment, the mRNA expression level is measured by RNA seq method.

In some embodiments, the expression level of said genes measured for example by quantitative RT-PCR are normalized by subtracting from each gene, the expression levels of housekeeping genes determined in the same experiment and the gene expression profile may correspond to the normalized gene expression level of one gene or to the sum of normalized gene expression level of at least two, three genes, preferably each of four genes selected from the group consisting of: *BCL2, CISH, PTGER2* and *DPP4* genes, more preferably each of 4, 5 or 6 genes selected from the group consisting of: *BCL2, CISH, PTGER2, DPP4, SOCS2* and *FLT3LG.*

In another embodiments, determining the gene expression profile comprises determining the expression level of said gene(s), in particular by RNA-seq or DNA microarray.

***In vitro* method for evaluating the therapeutic response of IL7 receptor modulator treatment during the course of the treatment.**

In a particular embodiment, the expression profile of at least one, preferably 2, 3, 4, 5, more preferably all combined of these genes is informative to monitor the antagonist or agonist effect of a compound (IL7R antagonist (e.g., anti-IL7R antagonist antibody or antigen binding fragment thereof) or IL7R agonist) in a patient and thus the efficacy of said treatment, during the course of the treatment.

The biomarkers according to the present disclosure make it possible to classify the patient as a responder of said treatment by determining the gene expression profile of at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in a patient sample and by determining whether the gene expression profile of said genes are high or low compared to a control value.

The present disclosure relates to an *in vitro* method for evaluating the therapeutic response of a human patient to treatment with an IL7R antagonist (e.g. anti-IL7 receptor antagonist antibody or antigen binding fragment thereof) by determining the gene expression profile of 1, 2, 3 or 4 genes selected from the group consisting of: human *BCL2, CISH, PTGER2* and *DPP4* genes, preferably at least 1, 2, 3, 4, 5 or 6 genes selected from the group consisting of: *BCL2, CISH, PTGER2, DPP4, SOCS* and *FLT3LG* genes in sample of a patient having received at least one dose of IL7R antagonist (e.g. anti-IL7 receptor antagonist antibody or antigen-binding fragment thereof), wherein a lower gene expression profile of said gene(s) in said patient sample as compared to a control value is indicative that patient is likely responsive to said antagonist treatment.

In another particular embodiment, the present disclosure relates to an *in vitro* method for evaluating the therapeutic response of a human patient to treatment with an IL7R agonist (e.g. IL7) by determining the gene expression profile of 1, 2, 3 or 4 genes selected from the group consisting of: human *BCL2, CISH, PTGER2* and *DPP4* genes, preferably at least 1, 2, 3, 4, 5 or 6 genes selected from the group consisting of: *BCL2, CISH, PTGER2, DPP4, SOCS* and *FLT3LG* genes in sample of a patient having received at least one dose of IL7R agonist, wherein a higher gene expression profile of said gene(s) in said patient sample as compared to a control value is indicative that patient is likely responsive to said agonist treatment.

The term "evaluating therapeutic response to treatment", as used herein, refers to an ability to assess whether the treatment of a patient is likely effective in (e.g., providing a measurable benefit or positive medical response to) the patient after some time of administration of the treatment. In other terms, according to the present disclosure, evaluating the response to a treatment refers to an ability to assess the effect of IL7R antagonist (e.g. anti-IL7R antagonist antibody or antigen-binding fragment thereof) or IL7R agonist in patient cells which can be correlated with the likely responsiveness of said patient to said treatment.

In particular, the antagonist or agonist effect of the compound in a patient and thus likely response to said antagonist or agonist treatment in a patient is evaluated by comparing the gene expression profile at least 1, 2, 3 or 4 genes selected from the group consisting of: *BCL2, CISH, PTGER2* and *DPP4* genes, preferably at least 1, 2, 3 ,4, 5 or 6 genes selected from the group consisting of: *BCL2, CISH, PTGER2, DPP4, SOCS2* and *FLT3LG* genes in a patient having received at least one dose of said antagonist to a control value.

According to the present method for evaluating therapeutic response, the sample is previously collected from a patient having received at least one dose of IL7R antagonist (e.g. anti-IL7R antagonist antibody or antigen-binding fragment thereof) or IL7R agonist.

The term "patient sample" means any biological sample derived from a patient. Examples of such samples include, tissues, cell samples, organs, biopsies, tumor sample etc. Preferred biological samples are peripheral blood mononuclear cells, tissue or tumor containing infiltrated immune cells.

In a particular embodiment, said sample comprises immune cells. Immune cells include lymphocytes, such as B cells and T cells and natural killer cells. Said immune cell can be obtained from a number of non-limiting sources, including peripheral blood mononuclear cells, bone marrow, lymph node tissue, cord blood, thymus tissue, tissue from a site of infection, ascites, pleural effusion, spleen tissue, and tumors, a unit of blood collected from a subject using any number of techniques known to the skilled person. Immune cells can be extracted from blood or derived from stem cells. In a preferred embodiment, said sample is tissue sample, preferably tumor tissue containing infiltrated immune cells from a cancer patient or a blood patient sample, more preferably human isolated peripheral blood mononuclear cells.

According to the present disclosure, a patient having received IL7R antagonist or agonist treatment refers to a patient having received at least one dose of IL7R antagonist (e.g., anti-IL7R antagonist antibody or antigen-binding fragment thereof) or IL7R agonist. The therapeutic response can be evaluated according to the present method, after each administration of a dose of IL7R antagonist or IL7R agonist throughout the course of treatment for monitoring the therapeutic response over time.

As used herein, the term "control value " may refer to the gene signature (*i.e.* gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes) in biological sample obtained from said patient at a different time, preferably prior to said administration of IL7R antagonist (e.g. anti-IL7R antagonist antibody or antigen-binding fragment thereof) dose or prior to any administration of antagonist dose or prior to any treatment of the condition or disease or obtained from patient cells cultured in vitro without said antagonist or agonist or with a compound (e.g. antibody) known to have no effect on IL7/IL7R pathway. The control value may alternatively be a predetermined value such as a threshold value, a standard value or a range obtained from other source than the patient's data. The control predetermined value may be established based upon comparative measurements between patients prior to said administration of antagonist (e.g. anti-IL7R antagonist antibody or antigen-binding fragment thereof) dose and patients having received said antagonist dose administration or upon comparative measurements between patient cells cultured in vitro with or without said antagonist or agonist or with a compound (e.g. antibody) known to have no effect on IL7/IL7R signaling pathway.

In a particular embodiment, according to the method for evaluating the therapeutic response of IL7 receptor modulator treatment during the course of the treatment of the present disclosure, the control value refers to the gene signature (*i.e.* gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes) in biological sample obtained from said patient at a different time, preferably prior to said administration of IL7R antagonist (e.g. anti-IL7R antagonist antibody or antigen-binding fragment thereof) dose or prior to any administration of antagonist dose or prior to any treatment of the condition or disease or the control value refers to a control predetermined value established based upon comparative measurements between patients prior to said administration of antagonist (e.g. anti-IL7R antagonist antibody or antigen-binding fragment thereof) dose and patients having received said antagonist dose administration.

In a preferred embodiment, the gene expression profile of said biomarkers is evaluated in a sample collected from a patient at least 1, 2, 3, or 7, preferably 10, 14, 21, 28 days after antibody dose administration, more particularly at least 30, 35, 40, 45, 50, 55, 60, 70, 80, 90, 100, 120 days after antibody dose administration.

A lower gene expression profile of said biomarkers in sample of patient having received at least one dose of IL7R antagonist (e.g. anti-IL7R antagonist antibody or antigen-binding fragment thereof) in comparison to control value correlates with antagonist effect of said antagonist (e.g. anti-IL7R antagonist antibody or antigen-binding fragment thereof) in patient and thus with therapeutic response of a patient to said IL7R antagonist (e.g. anti-IL7R antagonist antibody or antigen-binding fragment thereof) treatment.

Typically, the gene expression profile of said biomarkers in a patient sample is deemed to be lower than the control value if the log2 Fold change of the gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said patient to that of said control value is lower than at least 0.1, preferably 0.2, 0.3, 0.4, 0.5, 0.6, 0.7, 0.8, 0.9, more preferably 1, 2, 3, 4 again more preferably 5.

In particular, when the gene expression level is determined by quantitative RT-PCR, the gene expression profile of said biomarkers in a patient sample is deemed to be lower than the control value if the log2 Fold change of the gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said patient to that of said control value is lower than at least 0.4.

In another particular embodiment, when the gene expression level is determined by RNA-seq the gene expression profile of said biomarkers in a patient sample is deemed to be lower than the control value if the log2 Fold change of the gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said patient to that of said control value is lower than at least 0.1.

If after treatment with IL7R antagonist (e.g., anti-IL7R antagonist antibody or antigen-binding fragment thereof), gene expression profile of said biomarkers in sample of a patient having received at least one dose of antagonist is not lower than a control value, the treatment should be interrupted or modified, for example by changing said IL7R antagonist compound or by changing the concentration of said IL7R antagonist. The method for evaluating therapeutic response according to the present disclosure can indicate success or failure of a patient's treatment with IL7R antagonist (e.g. anti-IL7R antagonist antibody or antigen-binding fragment).

In another particular embodiment, a higher gene expression profile of said biomarkers in sample of patient having received at least one dose of IL7R agonist in comparison to control value correlates with agonist effect of said agonist in patient and thus with therapeutic response of a patient to said IL7R agonist treatment.

Typically, the gene expression profile of said biomarkers in a patient sample is deemed to be higher than the control value if the log2 Fold change of the gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said patient to that of said control value is higher than at least 0.1, preferably 0.2, 0.3, 0.4, 0.5, 0.6, 0.7, 0.8, 0.9 more preferably 1, 2, 3, again more preferably 4, even more preferably 5.

In particular, when the gene expression level is determined by quantitative RT-PCR, the gene expression profile of said biomarkers in a patient sample is deemed to be higher than the control value if the log2 Fold change of the gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said patient to that of said control value is higher than at least 0.4.

In another particular embodiment, when the gene expression level is determined by RNA-seq the gene expression profile of said biomarkers in a patient sample is deemed to be higher than the control value if the log2 Fold change of the gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said patient to that of said control value is higher than at least 0.1.

If after treatment with IL7R agonist, gene expression profile of said biomarkers in sample of a patient having received at least one dose of agonist is not higher than a control value, the treatment should be interrupted or modified, for example by changing said IL7R antagonist compound or by changing the concentration of said IL7R antagonist. The method for evaluating therapeutic response according to the present disclosure can indicate success or failure of a patient's treatment with IL7R agonist.

According to the present disclosure, said patient treated with IL7R antagonist (e.g. anti-IL7R antagonist antibody or antigen-binding fragment) suffers from a disorder or disease associated with an increase of the IL7R signaling pathway induced by IL7 (IL7/IL7R pathway).

Various pathological conditions are associated with abnormal high level of IL-7 and/or IL-7R. Said disorder associated with an increase of the IL7 signaling pathway induced by IL7 can be selected from the group consisting of an autoimmune disease, an inflammatory disease, an allergic disease, a cancer disease, an infectious disease, a respiratory disease, and a disease related to transplantation, involving the activation or proliferation of CD127 positive diseased cells, in particular an autoimmune disease or an inflammatory disease, involving the activation or proliferation of CD127 positive cells, more preferably a chronic inflammatory disease such as Sjogren's syndrome or inflammatory bowel disease such as ulcerative colitis.

Sjogren's disease is an autoimmune disease that primarily affects the exocrine glands resulting in their functional impairment. Sjogren's disease is an autoimmune disease that is characterized by inflammatory manifestations and can be categorized as inflammatory disease. The symptoms of Sjogren's syndrome include for examples dry eye and dry mouth. The Sjogren's syndrome often accompanies other immune system disorders such as rheumatoid arthritis, lupus, erythematosus, or progressive systemic sclerosis.

In a particular embodiment, said disorder associated with the increase of IL7R signaling pathway induced by IL7 is an infectious disease, preferably selected from the group consisting of chronic infectious disease, primary and secondary infectious disease.

In a particular embodiment, said disorder associated with the increase of IL7R signaling pathway induced by IL7 is autoimmune disease, preferably selected from the group consisting of: diabetes, multiple sclerosis, Sjogren's syndrome, lupus, psoriasis and atopic dermatis.

In another particular embodiment, said disease associated with an increase of the IL7R signaling pathway induced by IL7 is a chronic inflammatory disease, preferably selected from the group consisting of: rheumatoid arthritis, ankylosing spondylitis and inflammatory bowel disease.

In another particular embodiment, said disease associated with an increase of the IL7R signaling pathway induced by IL7 is allergic disease.

Said disease associated with an increase of the IL7R signaling pathway induced by IL7 can also be cancer such as hematological cancer, preferably selected from the group consisting of: T-cell acute lymphoblastic leukemia, B-cell acute lymphoblastic leukemia, chronic lymphocytic leukemia, T-cell prolymphocytic leukemia and Hodgkin's lymphoma.

In a preferred embodiment, said disorder associated with an increase of the IL7R signaling pathway induced by IL7 is Sjogren's syndrome or inflammatory bowel disease such as ulcerative colitis or Crohn's disease.

According to the present disclosure, said patient treated with IL7R agonist suffers from a disorder or disease associated with impaired healthy T-cell activity, preferably that can be rescued through IL7R signaling pathway induced by IL7, preferably a cancer disease or an infectious disease which does not itself depend on the IL7R pathway for its development. In a particular embodiment, said patient treated with IL7R agonist suffers from a disorder or disease wherein enhancement of immune response is required such as immunotherapy in a cancer disease or an infectious disease.

In another particular embodiment, the present disclosure relates to the therapeutic use of an IL7R antagonist (e.g. an anti-IL7 receptor antagonist antibody or antigen-binding fragment thereof) or agonist in a human patient in need thereof wherein said antagonist, in particular said antibody or agonist is administered to said patient after the patient has been administered with at least one dose of IL7R antagonist (e.g. an anti-IL7 receptor antagonist antibody or antigen-binding fragment thereof) or IL7R agonist and when said patient is evaluated as likely responsive to said antagonist or agonist treatment in a method as described above.

The present invention relates also to a method of treating a disease associated with an increase of IL7R signalling pathway induced by IL7 or a disease associated with impaired healthy T-cell activity as described above in a human patient in need thereof comprising:
(a) providing a sample previously collected from said patient having received at least one dose of IL7R antagonist (e.g., an anti-IL7 receptor antagonist antibody or antigen-binding fragment thereof) or IL7R agonist,
(b) determining whether the gene expression profile of at least 1, 2, 3, or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4,* FLT3LG and SOCS2 is lower or higher as compared to a control value, wherein a lower or higher gene expression profile of said genes is indicative that said patient is likely responsive to said IL7R antagonist (e.g. an anti-IL7 receptor antagonist antibody or antigen-binding fragment thereof) or IL7R agonist treatment respectively,
(c) re-administering to said patient who has been evaluated as likely responsive to said antagonist or agonist treatment a therapeutically effective amount of IL7R antagonist (e.g. anti-IL7 receptor antagonist antibody or antigen-binding fragment thereof) or IL7R agonist.

In another terms, the present disclosure relates to a method for evaluating therapeutic response of an IL7R antagonist treatment in a human patient in need thereof suffering from a disease associated with an increase of IL7R signaling pathway induced by IL7 as described above comprising:
(a) providing a sample collected from said patient at a first or earlier point of time,
(b) providing a sample collected from said patient at a second or subsequent point of time,
(b) determining whether the gene expression profile of at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, FLT3LG* and *SOCS2* genes in said patient sample collected at a second or subsequent point of time is lower as compared to the patient sample collected at a first or earlier point of time, wherein a lower gene expression profile is indicative that said patient is likely responsive to said treatment,
(c) re-administering to said patient who has been evaluated as likely responsive to said antagonist treatment a therapeutically effective amount of IL7R antagonist (e.g., anti-IL7 receptor antagonist antibody or antigen-binding fragment thereof).

The present disclosure also relates to a method for evaluating therapeutic response of an IL7R agonist treatment in a human patient in need thereof suffering from suffers from a disorder or disease associated with impaired healthy T-cell activity, preferably that can be rescued through IL7R signaling pathway induced by IL7, comprising:
(a) providing a sample collected from said patient at a first or earlier point of time,
(b) providing a sample collected from said patient at a second or subsequent point of time,
(b) determining whether the gene expression profile of at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, FLT3LG* and *SOCS2* genes in said patient sample collected at a second or subsequent point of time is higher as compared to said patient sample collected at a first or earlier point of time, wherein a higher gene expression profile is indicative that said patient is likely responsive to said treatment,
(c) re-administering to said patient who has been evaluated as likely responsive to said agonist treatment a therapeutically effective amount of IL7R agonist.

The present disclosure relates to the use of IL7R antagonist (e.g. an anti-IL7 receptor antagonist antibody or antigen-binding fragment thereof as described above) or IL7R agonist in the manufacture of a medicament for the treatment of disease associated with the IL7R signaling pathway induced by IL7 or a disease associated with impaired healthy T-cell activity in a human patient in need thereof wherein said antagonist or agonist is administered to said patient after the patient has been administered with at least one dose of said IL7R antagonist (e.g. an anti-IL7 receptor antagonist antibody or antigen-binding fragment thereof as described above) or IL7R agonist and when said patient is evaluated as likely responsive to said antagonist or agonist treatment in a method as described above.

In a particular embodiment, said disorder or disease associated with an increase of the IL7 signaling pathway induced by IL7 treated with IL7R antagonist is selected from the group consisting of an autoimmune disease, an inflammatory disease, an allergic disease, a cancer disease, an infectious disease, a respiratory disease, and a disease related to transplantation, involving the activation or proliferation of CD127 positive diseased cells, in particular an autoimmune disease or an inflammatory disease, involving the activation or proliferation of CD127 positive cells, more preferably a chronic inflammatory disease such as Sjogren's syndrome or inflammatory bowel disease such as ulcerative colitis, preferably diabetes, sarcoidosis, cancer, autoimmune disease preferably selected from the group consisting of: multiple sclerosis, Sjogren's syndrome, lupus, psoriasis and atopic dermatis, or chronic inflammatory disease, preferably selected from the group consisting of: rheumatoid arthritis, ankylosing spondylitis and inflammatory bowel disease, preferably ulcerative colitis or Crohn's disease.

In a particular embodiment, said IL7R antagonist is an anti-IL7R antagonist antibody or antigen binding fragment thereof according to the present disclosure comprises: (a) a variable heavy chain comprising three CDRs wherein VHCDR1 comprises or consists of the amino acid sequence of SEQ ID NO: 1, VHCDR2 comprises or consists of the amino acid sequence of SEQ ID NO: 2, and VHCDR3 comprises or consists of the amino acid sequence of SEQ ID NO: 3 or 11 and; (b) a variable light chain comprising three CDRs wherein VLCDR1 comprises or consists of the amino acid sequence of SEQ ID NO: 4 or 12, VLCDR2 comprises or consists of the amino acid sequence of SEQ ID NO: 5 or 13 and VLCDR3 comprises or consists of the amino acid sequence of SEQ ID NO: 6.

In a preferred embodiment, said anti-IL7 receptor antagonist antibody or antigen binding fragment thereof comprises: (a) a variable heavy chain comprising three CDRs wherein VHCDR1 comprises or consists of the amino acid sequence of SEQ ID NO: 1, VHCDR2 comprises or consists of the amino acid sequence of SEQ ID NO: 2, and VHCDR3 comprises or consists of the amino acid sequence of SEQ ID NO: 3 and; (b) a variable light chain comprising three CDRs wherein VLCDR1 comprises or consists of the amino acid sequence of SEQ ID NO: 4, VLCDR2 comprises or consists of the amino acid sequence of SEQ ID NO: 5 and VLCDR3 comprises or consists of the amino acid sequence of SEQ ID NO: 6.

In a particular embodiment, said anti-IL7R antagonist antibody or antigen binding fragment thereof comprises a heavy chain variable domain comprising or consisting of the amino acid sequence selected from the group consisting of: SEQ ID NO: 14-17, and a light chain variable domain comprising or consisting of the amino acid sequence selected from the group consisting of SEQ ID NO: 18-21, preferably, said anti-IL7R antagonist is N13B2h3 antibody or antigen binding fragment thereof as described in WO2015/189302 and comprises a heavy chain variable domain comprising or consisting of the amino acid sequence of: SEQ ID NO: 17, and a light chain variable domain comprising or consisting of the amino acid sequence of SEQ ID NO: 21, more preferably said antibody or antigen binding fragment thereof comprises a full heavy chain comprising or consisting of SEQ ID NO: 22 and a full light chain comprising or consisting of SEQ ID NO: 23.

In another particular embodiment, said anti-IL7R antagonist antibody or antigen binding fragment thereof comprises a heavy chain variable domain comprising or consisting of the amino acid sequence SEQ ID NO: 7, and a light chain variable domain comprising or consisting of the amino acid sequence selected from the group consisting of SEQ ID NO: 8, 24-26.

In a more preferred embodiment, said anti-IL7R antagonist antibody or antigen binding fragment thereof comprises a heavy chain variable domain comprising or consisting of the amino acid sequence SEQ ID NO: 7 and a light chain variable domain comprising or consisting of the amino acid sequence SEQ ID NO: 8, preferably said antibody or antigen binding fragment thereof comprises a full heavy chain comprising or consisting of SEQ ID NO: 9 and a full light chain comprising or consisting of SEQ ID NO: 10.

In the context of the present disclosure, the term "treating" or "treatment", as used herein, means reversing, alleviating, inhibiting the progress of, or preventing the disorder or condition to which such term applies, or reversing, alleviating, inhibiting the progress of, or preventing one or more symptoms of the disorder or condition to which such term applies.

As used herein, a "therapeutically effective amount" or an "effective amount" means the amount of a composition that, when administered to a subject for treating a state, disorder or condition is sufficient to effect a treatment. The therapeutically effective amount will vary depending on the compound, formulation or composition, the disease and its severity and the age, weight, physical condition and responsiveness of the subject to be treated.

The IL7R antagonist (e.g., anti-IL7R antagonist antibody or antigen binding fragment thereof) or IL7R agonist described herein may be administered by any means known to those skilled in the art, including, without limitation, intravenously, orally, intra-tumoral, intra-lesional, intradermal, topical, intraperitoneal, intramuscular, parenteral, subcutaneous and topical administration. Thus, the compositions may be formulated as an injectable, topical, or ingestible formulation. Administration of the compounds or therapeutic agents to a subject in accordance with the present disclosure may exhibit beneficial effects in a dose-dependent manner. Thus, within broad limits, administration of larger quantities of the compositions is expected to achieve increased beneficial biological effects than administration of a smaller amount. Moreover, efficacy is also contemplated at dosages below the level at which toxicity is seen.

It will be appreciated that the specific dosage of IL7R antagonist or agonist administered in any given case will be adjusted in accordance with the composition being administered, the volume of the composition that can be effectively delivered to the site of administration, the disease to be treated or inhibited, the condition of the subject, and other relevant medical factors that may modify the activity of the compositions or the response of the subject, as is well known by those skilled in the art.

For example, the specific dose of IL7R antagonist or agonist for a particular subject depends on age, body weight, general state of health, diet, the timing and mode of administration, the rate of excretion, medicaments used in combination and the severity of the particular disorder to which the therapy is applied. Dosages for a given patient can be determined using conventional considerations, e.g., by customary comparison of the differential activities of the compositions described herein and of a known agent, such as by means of an appropriate conventional pharmacological protocol. The compositions can be given in a single dose schedule, or in a multiple dose schedule.

Suitable dosage ranges for IL7R antagonist (e.g., anti-IL7R antagonist antibody or antigen binding fragment thereof) or IL7R agonist may be of the order of several hundred micrograms of the agent with a range from about 0.001 to 100 mg/kg, preferably with the range from about 0.1 to 20 mg/kg, more preferably from about 1 to 12 mg/kg, again more preferably 8-12 mg/kg. For instance, a flat dose of 400, 450, 500, 600, 700, 850, 900 mg can be used.

**A method for assessing the likelihood of a therapeutic response in a human patient to IL7R modulator treatment prior to said treatment.**

The combined expression profile of said biomarkers may be also informative of the status of the patient who, before any treatment, can be classified as likely to respond, and for whom a treatment with an antagonist (e.g., anti-IL7 receptor antagonist antibody or antigen-binding fragment thereof) or IL7R agonist described herein is recommended. For instance, the assay can comprise culturing a sample of said patient before the treatment in presence of the IL7R antagonist or agonist in order to assess its biological response.

Thus, in another particular embodiment, the present disclosure relates to a method for assessing the likelihood of therapeutic response in a human patient to IL7R antagonist (e.g. anti-IL7 receptor antagonist antibody or antigen-binding fragment thereof) or agonist treatment prior to said antagonist or agonist treatment comprising the steps of:
a) culturing in presence of IL7 receptor antagonist and/or IL7R agonist a sample previously collected from said patient prior to said antagonist or agonist treatment,
b) determining the gene expression profile of at least 1, 2, 3 or 4 genes selected from the group consisting of: *BCL2, CISH, PTGER2* and *DPP4* genes, preferably at least 1, 2, 3, 4, 5 or 6 genes selected from the group consisting of *BCL2, CISH, PTGER2, DPP4, SOCS2* and *FLT3LG* genes, wherein a lower or higher gene expression profile of said genes in cultured sample as compared to a control value is indicative that said patient is likely responsive to the antagonist or agonist treatment respectively.

The term "assessing the likelihood of therapeutic response" as used herein, refers to an ability to assess whether the patient will respond to the treatment with an IL7R antagonist (e.g. anti-IL7 receptor antagonist antibody or antigen-binding fragment thereof) or IL7R agonist, in another term whether the antagonist or agonist treatment will be effective in (e.g., providing a measurable benefit or positive medical response to) the patient in advance of therapy. Such ability to assess whether patient will respond to the treatment with said antagonist or agonist is typically exercised before antagonist or agonist treatment has begun in the patient.

According to the present method of assessing the likelihood of therapeutic response, the sample is previously collected from a patient before antagonist or agonist treatment begins. Said sample is preferably immune cells, more preferably peripheral blood mononuclear cells collected from said patient before treatment with said antagonist or agonist as described above.

In a particular embodiment, said cells are immune cells. Immune cells include lymphocytes, such as B cells and T cells, natural killer cells. Said immune cell can be obtained from a number of non-limiting sources, including peripheral blood mononuclear cells, bone marrow, lymph node tissue, cord blood, thymus tissue, tissue from a site of infection, ascites, pleural effusion, spleen tissue, and tumors, a unit of blood collected from a subject using any number of techniques known to the skilled person. Immune cells can be extracted from blood or derived from stem cells. In a preferred embodiment, said sample is tissue sample, preferably tumor tissue containing infiltrated immune cells from a cancer patient or a blood patient sample, more preferably human isolated peripheral blood mononuclear cells.

Said sample is thereafter cultured *in vitro* in presence of an IL7R antagonist (e.g., anti-IL7 receptor antagonist antibody or antigen-binding fragment thereof) and/or IL7R agonist.

Sample may be cultured in culture media under culture condition well-known in the art to be expanded before to be harvested for gene expression level analysis. In a preferred embodiment, said sample is cultured during at least 3, preferably 2, 4, 5 or 6 hours at 37°C before to be harvested for further analysis.

Numerous culture mediums are available commercially and are well-known to the person skilled in the art. This medium may be a minimum medium particularly comprising mineral salts, amino acids, vitamins and a carbon source essential to cells and a buffer system for regulating pH. The basal medium able to be used in the method according to the invention includes, for example, but are not limited to, DMEM/F12 medium, DMEM medium, RPMI medium, Ham's F12 medium, IMDM medium and KnockOut^{™} DMEM medium (Life Technologies). Said medium may comprise interleukine-7, preferably human recombinant interleukine-7.

In a particular embodiment, in said method for assessing the likelihood of therapeutic response in a human patient to IL7R antagonist, said sample previously collected from said patient prior to said antagonist treatment is cultured in step a) in presence of IL7 receptor antagonist (e.g., anti-IL7 receptor antagonist antibody or antigen-binding fragment thereof) and IL7 cytokine, preferably human recombinant interleukine-7 to allow cell expansion.

Said culture medium comprises an IL7R antagonist, preferably an anti-IL7 receptor antagonist antibody or antigen binding fragment thereof, more preferably at a concentration comprised between 1 and 10 000 ng/mL, preferably 500 and 10 000 ng/mL, more preferably between 1000 and 10 000 ng/mL.

Said culture medium comprises an IL7R agonist, preferably at a concentration comprised between 1 and 10 000 ng/mL, preferably 500 and 10 000 ng/mL, more preferably between 1000 and 10 000 ng/mL.

After cell culture step with an IL7R antagonist (e.g. anti-IL7 receptor antagonist antibody or antigen-binding fragment thereof) and/or IL7R agonist, said cells are harvested and the gene expression profile of at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes is determined as described above in said cultured cells.

The method further comprises determining whether the gene expression profile of said genes is high or low compared to a control value. For instance, a lower or higher gene expression profile of at least one of said genes compared to the control value may be indicative of a patient being likely to respond to the antagonist or agonist treatment respectively

In a particular embodiment, according to the method for assessing the likelihood of a therapeutic response in a human patient of the present disclosure, the control value can be the gene expression profile of said gene(s) in patient cells cultured *in vitro* without said antagonist or agonist or with a compound (e.g. antibody) known to have no effect on IL7/IL7R pathway or a control predetermined value established upon comparative measurements between patient cells cultured in vitro with or without said antagonist or agonist or with a compound (e.g. antibody) known to have no effect on IL7/IL7R pathway.

A lower gene expression profile of at least one of said biomarkers in patient cells cultured *in vitro* with IL7R antagonist (e.g. anti-IL7 receptor antagonist antibody or antigen-binding fragment thereof) in comparison to a control value is indicative of IL7R antagonist effect in said patient cells and thus correlates with likelihood of therapeutic response to the IL7R antagonist treatment in a patient.

Typically, the gene expression profile of said gene(s) in a patient sample cultured *in vitro* with IL7R antagonist (e.g. anti-IL7R antagonist antibody or antigen binding fragment thereof) is deemed to be lower than a control value if the log2 Fold change of the gene expression profile of the gene(s) in said patient to that of said control value is lower than at least 0.1, preferably 0.2, 0.3, 0.4, 0.5, 0.6,0.7, 0.8, 0.9, more preferably 1, 2, 3, 4, again more preferably 5.

In particular, when the gene expression level is determined by quantitative RT-PCR, the gene expression profile of said biomarkers in a patient sample is deemed to be lower than the control value if the log2 Fold change of the gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said patient to that of said control value is lower than at least 0.4.

In another particular embodiment, when the gene expression level is determined by RNA-seq the gene expression profile of said biomarkers in a patient sample is deemed to be lower than the control value if the log2 Fold change of the gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said patient to that of said control value is lower than at least 0.1.

In another particular embodiment, a higher gene expression profile of at least one of said biomarkers in patient cells cultured *in vitro* with IL7R agonist in comparison to a control value is indicative of IL7R agonist effect in said patient cells and thus correlates with likelihood of therapeutic response to the IL7R agonist treatment in a patient.

Typically, the gene expression profile of said gene(s) in a patient sample cultured *in vitro* with IL7R agonist is deemed to be higher than a control value if the log2 Fold change of the gene expression profile of the gene(s) in said patient to that of said control value is higher than at least 0.1, preferably 0.2, 0.3, 0.4, 0.5, 0.6, 0.7, 0.8, 0.9 more preferably 1, 2, 3, 4, again more preferably 5.

In particular, when the gene expression level is determined by quantitative RT-PCR, the gene expression profile of said biomarkers in a patient sample is deemed to be higher than the control value if the log2 Fold change of the gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said patient to that of said control value is higher than at least 0.4.

In another particular embodiment, when the gene expression level is determined by RNA-seq the gene expression profile of said biomarkers in a patient sample is deemed to be higher than the control value if the log2 Fold change of the gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said patient to that of said control value is higher than at least 0.1.

According to the present disclosure, said patient treated with IL7R antagonist (e.g. anti-IL7 receptor antagonist antibody or antigen-binding fragment thereof) suffers from a disorder or disease associated with an increase of the IL7R signaling pathway induced by IL7, preferably selected from the group consisting of: an autoimmune disease, an inflammatory disease, an allergic disease, a cancer disease, an infectious disease, a respiratory disease, and a disease related to transplantation, involving the activation or proliferation of CD127 positive diseased cells, in particular an autoimmune disease or an inflammatory disease, involving the activation or proliferation of CD127 positive cells, more preferably a chronic inflammatory disease such as Sjogren's syndrome or inflammatory bowel disease such as ulcerative colitis as described above.

According to the present disclosure, said patient treating with IL7R agonist suffers from a disorder or disease associated with impaired healthy T-cell activity, preferably a cancer disease or an infectious disease which does not itself depend on the IL7R pathway for its development as described above.

The present disclosure relates also to the therapeutic use of IL7R antagonist (e.g. anti-IL7 receptor antagonist antibody or antigen-binding fragment thereof) or IL7R agonist in a human patient wherein said patient was previously assessed to be likely responsive to said antagonist or agonist treatment prior to said treatment in the method as described above. The present disclosure also relates to a method of treating a disease associated with an increase of IL7R pathway induced by IL7 in a human patient in need thereof with IL7R antagonist (e.g., anti-IL7 receptor antagonist antibody or antigen-binding fragment thereof) comprising:
(a) culturing in presence of IL7R antagonist (e.g., anti-IL7 receptor antagonist antibody or antigen-binding fragment thereof) a sample previously collected from a patient prior to said treatment, and
(b) determining whether the gene expression profile of at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, FLT3LG* and *SOCS2* genes in said sample is lower as compared to a control value, wherein a lower gene expression profile in said patient sample as compared to a control value is indicative that said patient is likely responsive to said treatment,
(c) administering a therapeutically effective amount of IL7R antagonist (e.g., anti-IL7 receptor antagonist antibody or antigen-binding fragment thereof) to said patient assessed to be likely responsive to said treatment.

The present disclosure also relates to a method of treating a disease associated with impaired healthy T-cell activity as described above in a human patient in need thereof with IL7R agonist comprising:
(a) culturing with IL7R agonist a sample previously collected from a patient prior to said treatment, and
(b) determining whether the gene expression profile of at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, FLT3LG* and *SOCS2* genes in said sample is higher as compared to a control value, wherein a higher gene expression profile in said patient sample as compared to a control value is indicative that said patient is likely responsive to said treatment,
(c) administering a therapeutically effective amount of IL7R agonist to said patient assessed to be likely responsive to said treatment.

The present disclosure also relates to the use of IL7R antagonist (e.g. anti-IL7 receptor antagonist antibody or antigen-binding fragment thereof) or IL7R agonist in the manufacture of a medicament for the treatment of disease associated with the IL7/IL7R pathway or a disease associated with impaired healthy T-cell activity as described above in a human patient in need thereof wherein said patient was previously assessed to be likely responsive to said antagonist or agonist treatment in the method as described above.

In a particular embodiment, said anti-IL7R antagonist is N13B2h1, N13B2h2 or N13B2h3 antibody or antigen binding fragment thereof as described in WO2015/189302 or N13B2-hVL3, N13B2-hVL4, N13B2-hVL5 or N13B2-hVL6 antibody or antigen binding fragment thereof as described in WO2018/104483 and comprises: (a) a variable heavy chain comprising three CDRs wherein VHCDR1 comprises or consists of the amino acid sequence of SEQ ID NO: 1, VHCDR2 comprises or consists of the amino acid sequence of SEQ ID NO: 2, and VHCDR3 comprises or consists of the amino acid sequence of SEQ ID NO: 3 or 11 and; (b) a variable light chain comprising three CDRs wherein VLCDR1 comprises or consists of the amino acid sequence of SEQ ID NO: 4 or 12, VLCDR2 comprises or consists of the amino acid sequence of SEQ ID NO: 5 or 13 and VLCDR3 comprises or consists of the amino acid sequence of SEQ ID NO: 6

In a preferred embodiment, said anti-IL7 receptor antagonist antibody or antigen-binding fragment thereof is N13B2h3 antibody or antigen binding fragment thereof as described in WO2015/189302 or N13B2-hVL3, N13B2-hVL4, N13B2-hVL5 or N13B2-hVL6 antibody or antigen binding fragment thereof as described in WO2018/104483, preferably N13B2-hVL6 antibody or antigen-binding fragment thereof and comprises: (a) a variable heavy chain comprising three CDRs wherein VHCDR1 comprises or consists of the amino acid sequence of SEQ ID NO: 1, VHCDR2 comprises or consists of the amino acid sequence of SEQ ID NO: 2, and VHCDR3 comprises or consists of the amino acid sequence of SEQ ID NO: 3 and; (b) a variable light chain comprising three CDRs wherein VLCDR1 comprises or consists of the amino acid sequence of SEQ ID NO: 4, VLCDR2 comprises or consists of the amino acid sequence of SEQ ID NO: 5 and VLCDR3 comprises or consists of the amino acid sequence of SEQ ID NO: 6.

In a particular embodiment, said anti-IL7R antagonist antibody or antigen-binding fragment thereof comprises a heavy chain variable domain comprising or consisting of the amino acid sequence selected from the group consisting of: SEQ ID NO: 14-17, and a light chain variable domain comprising or consisting of the amino acid sequence selected from the group consisting of SEQ ID NO: 18-21, preferably, said anti-IL7R antagonist is N13B2h3 antibody or antigen binding fragment thereof as described in WO2015/189302 and comprises a heavy chain variable domain comprising or consisting of the amino acid sequence of: SEQ ID NO: 17, and a light chain variable domain comprising or consisting of the amino acid sequence of SEQ ID NO: 21 more preferably said antibody or antigen binding fragment thereof comprises a full heavy chain comprising or consisting of SEQ ID NO: 22 and a full light chain comprising or consisting of SEQ ID NO: 23.

In another particular embodiment, said anti-IL7R antagonist is N13B2-hVL3, N13B2-hVL4, N13B2-hVL5 or N13B2-hVL6 antibody or antigen binding fragment thereof as described in WO2018/104483 and comprises a heavy chain variable domain comprising or consisting of the amino acid sequence SEQ ID NO: 7, and a light chain variable domain comprising or consisting of the amino acid sequence selected from the group consisting of SEQ ID NO: 8, 24-26.

In a more preferred embodiment, said anti-IL7R antagonist antibody or antigen-binding fragment thereof comprises a heavy chain variable domain comprising or consisting of the amino acid sequence SEQ ID NO: 7 and a light chain variable domain comprising or consisting of the amino acid sequence SEQ ID NO: 8, preferably said antibody or antigen-binding fragment thereof comprises a full heavy chain comprising or consisting of SEQ ID NO: 9 and a full light chain comprising or consisting of SEQ ID NO: 10.

The antagonist, preferably antibody or antigen-binding fragment thereof described herein or agonist may be administered by any means known to those skilled in the art, as described above.

Suitable dosage ranges for IL7R antagonist, preferably anti-IL7R antagonist antibody or antigen-binding fragment thereof or IL7R agonist may be of the order of several hundred micrograms of the agent with a range from about 0.001 to 100 mg/kg, preferably with the range from about 0.1 to 20 mg/kg, more preferably from about 1 to 12 mg/kg, again more preferably 8-12 mg/kg. For instance, a flat dose of 400, 450, 500, 600, 700, 850, 900 mg can be used.

### Method for selecting an IL7R modulator compound

In another particular embodiment, the present disclosure relates to an *in vitro* method for selecting an IL7R antagonist compound, preferably IL7Rα antagonist compound likely to be effective in the treatment of disease associated with an increase of IL7R signaling pathway induced by IL7 as described above comprising the steps of:
a) culturing human cells with said compound,
b) determining the gene expression profile of at least 1, 2, 3 or 4 genes selected from the group consisting of *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said cells, and
c) selecting a compound that induces a lower gene expression profile of said gene(s) in said cells as compared to a control value.

In another particular embodiment, the present disclosure relates to an *in vitro* method for selecting an IL7R agonist compound likely to be effective in the treatment of disease associated with impaired healthy T-cell activity as described above comprising the steps of:
a) culturing human cells with said agonist compound,
b) determining the gene expression profile of at least 1, 2, 3 or 4 genes selected from the group consisting of *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said cells, and
c) selecting a compound that induces a higher gene expression profile of said gene(s) respectively in said cells as compared to a control value.

According to the present method for selecting an IL7R antagonist or agonist compound, said control value can be the gene expression profile of said gene(s) in cells cultured without a compound or which is known to have no effect on IL7/IL7R signaling pathway.

In a preferred embodiment said compound is an anti-IL7R antagonist antibody or antigen-binding fragment thereof.

Cells that can be used for selecting said compound can be immortalized cell lines expressing CD127, such as T-cell line, for example Jurkat cells, or cells collected from a donor, preferably a human healthy donor.

In a particular embodiment, said cells are immune cells that include lymphocytes, in particular T cells, natural killer cells. Said immune cell can be obtained from a number of non-limiting sources, including peripheral blood mononuclear cells, bone marrow, lymph node tissue, cord blood, thymus tissue, tissue from a site of infection, ascites, pleural effusion, spleen tissue, and tumors, a unit of blood collected from a subject using any number of techniques known to the skilled person. Immune cells can be extracted from blood or derived from stem cells. In a preferred embodiment, said cells are peripheral blood mononuclear cells.

It may be considered that an antagonist is a suitable IL7-R antagonist, preferably anti-IL7R antagonist antibody or antigen-binding fragment thereof and is likely to be effective in the treatment of disease associated with an increase of IL7R signaling pathway induced by IL7 when it induces a lower gene expression profile of at least 1, 2, 3 or 4 genes selected from the group consisting of : *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in cell cultured with said compound as compared to a control value, preferably if log2 Fold change of the gene expression profile of at least 1, 2, 3 or 4 genes selected from the group consisting of: *BCL2, CISH, PTGER2, DPP4,* preferably 1, 2, 3, 4, 5 or 6 genes selected from the group consisting of: *BCL2, CISH, PTGER2, DPP4, SOCS2* and *FLT3LG* genes in said cells cultured with said compound to that of said control value is lower than at least 0.1, preferably 0.2, 0.3, 0.4, 0.5, 0.6, 0.7, 0.8, 0.9, more preferably 1, 2, 3, 4, , even more preferably 5.

In particular, when the gene expression level is determined by quantitative RT-PCR, the gene expression profile of said biomarkers in a patient sample is deemed to be lower than the control value if the log2 Fold change of the gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said patient to that of said control value is lower than at least 0.4.

In another particular embodiment, when the gene expression level is determined by RNA-seq the gene expression profile of said biomarkers in a patient sample is deemed to be lower than the control value if the log2 Fold change of the gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said patient to that of said control value is lower than at least 0.1.

Otherwise, it may be considered that an agonist is a suitable IL7R agonist and is likely to be effective in the treatment of disease associated with impaired healthy T-cell activity as described above when it induces a higher gene expression profile of at least 1, 2, 3 or 4 genes selected from the group consisting of : *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in cell cultured with said compound as compared to a control value, preferably if the log2 Fold change of the gene expression profile of at least 1, 2, 3 or 4 genes selected from the group consisting of: *BCL2, CISH, PTGER2, DPP4,* preferably 1, 2, 3, 4, 5 or 6 genes selected from the group consisting of: *BCL2, CISH, PTGER2, DPP4, SOCS2* and *FLT3LG* genes in said cells cultured with said compound to that of said control value is higher than 0.1, preferably 0.2, 0.3, 0.4, 0.5, 0.6, 0.7, 0.8,0.9, more preferably 1, 2, 3, 4, even more preferably 5

In particular, when the gene expression level is determined by quantitative RT-PCR, the gene expression profile of said biomarkers in a patient sample is deemed to be higher than the control value if the log2 Fold change of the gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said patient to that of said control value is higher than at least 0.4.

In another particular embodiment, when the gene expression level is determined by RNA-seq the gene expression profile of said biomarkers in a patient sample is deemed to be higher than the control value if the log2 Fold change of the gene expression profile at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes in said patient to that of said control value is higher than at least 0.1.

### Kit

In another aspect, the present disclosure relates to a kit, preferably for use in an in vitro method for evaluating the therapeutic response of an IL7R antagonist or an IL7R agonist or an in vitro method for assessing the likelihood of a therapeutic response in a human patient to an IL7R antagonist or agonist comprising or consisting of a set of reagents that specifically detects the gene expression profile of at least 1, 2, 3 or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes.

Preferably, the kit comprises containers each comprising one or more compounds at a concentration or in an amount that facilitates the reconstitution and/or the use of a set of reagents that specifically detects the gene expression profile of at least 1, 2, 3, or 4 genes selected from the group consisting of: *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes and the implementation of the method according to the disclosure.

In particular, the kit contains primer pair and or probes specific of at least 1, 2, 3, or 4 genes selected from the group consisting of: of *BCL2, CISH, PTGER2* and *DPP4* genes, preferably at least 1, 2, 3, 4, 5 or 6 genes selected from the group consisting of: *BCL2, CISH, PTGER2, DPP4, SOCS2* and *FLT3LG* genes.

The kit may also comprise instructions indicating the methods for preparing and/or using the reagents to determine the expression level of said genes according to the methods of the disclosure.

The present invention also relates to the use of the kit for a method according to the present disclosure.

The invention will now be exemplified with the following examples, which are not limitative, with reference to the attached Figures.

### FIGURE LEGENDS

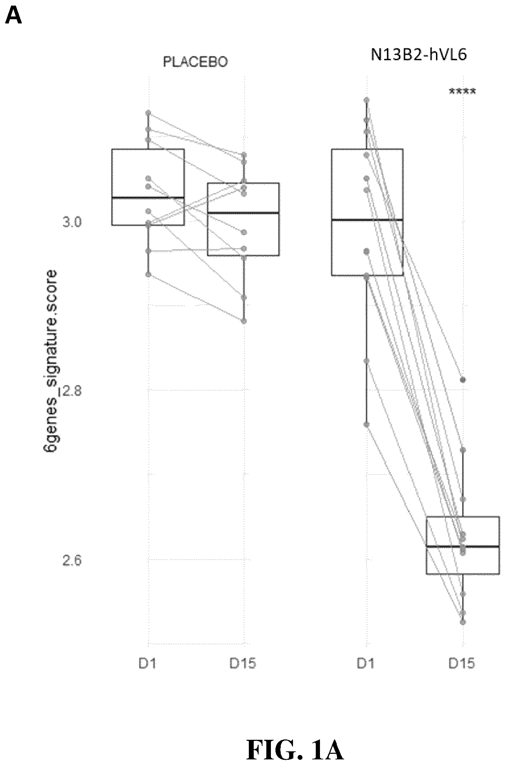

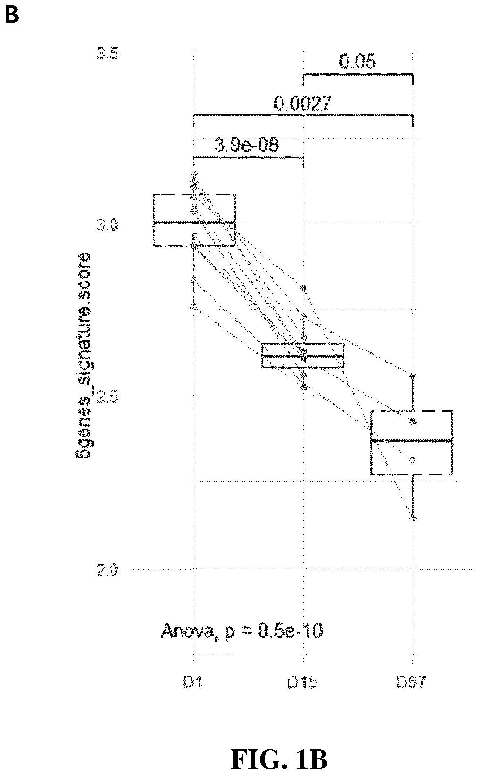

**Figure 1****:** Boxplot representation of the 6 genes ssGSEA signature (BCL2, CISH, PTGER2, DPP4, FLT3LG and SOCS2) on the A) N13B2-hVL6 or placebo D1 and D15 paired samples and B) on D1, D15 and D57 paired samples from N13B2-hVL6 treated samples with RNAseq method. A significant and high decrease of the signature related to the IL7/IL7R pathway was observed for N13B2-hVL6 with these 6 genes.

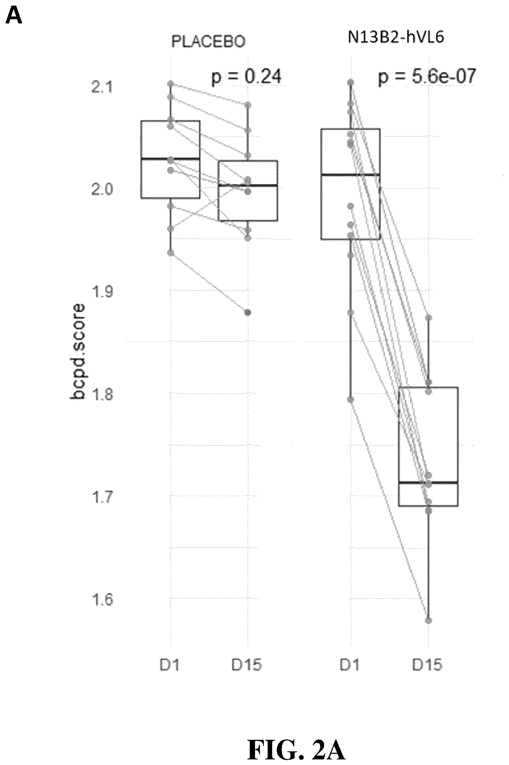

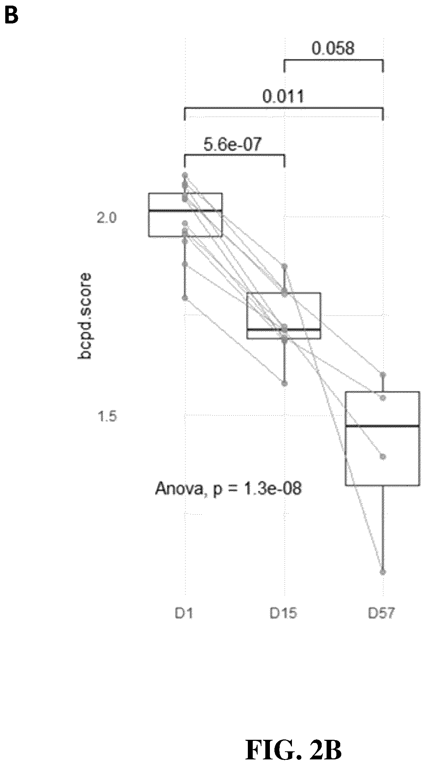

**Figure 2****:** Boxplot representation of the new 4 genes ssGSEA signature (BCL2, CISH, PTGER2, DPP4) on the A) N13B2-hVL6 or placebo D1 and D15 paired samples and B) on D1, D15 and D57 paired samples from N13B2-hVL6 treated samples with RNAseq method. A significant and high decrease of the signature related to the IL7/IL7R pathway was observed for N13B2-hVL6 with these 4 genes.
**Figure 3****:** Boxplot representation of the new 4 genes signature (BCL2, CISH, PTGER2, DPP4) on the N13B2-hVL6 or placebo D1 and D15 paired samples with RT-qPCR method. A significant and high decrease of the signature related to the IL7/IL7R pathway was observed for N13B2-hVL6 with these 4 genes.

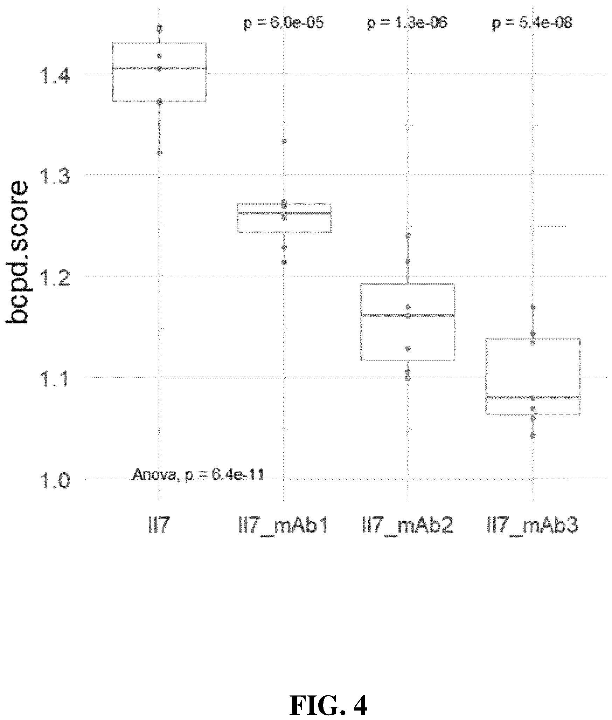

**Figure 4****:** Boxplot representation of the 4 genes ssGSEA signature with *BCL2, CISH, PTGER2, DPP4* gene expression calculated with GSVA R package on RNAseq sequencing of human PBMCs preincubated with 3 anti-human IL7Ra mAb (mAb1 : N13B2-hVL6 (disclosed in WO2018/104483), mAb2 : MD707-13 (anti-site 1 antibody disclosed in WO2013/056984), mAb3 : GSKWO2011 (1A11 disclosed in GSK patent WO2011/094259)) and stimulated 3h with recombinant IL7. A significant and high decrease of the ssGSEA signature related to the IL7/IL7R pathway was observed for the 3 antibodies with these 4 genes.

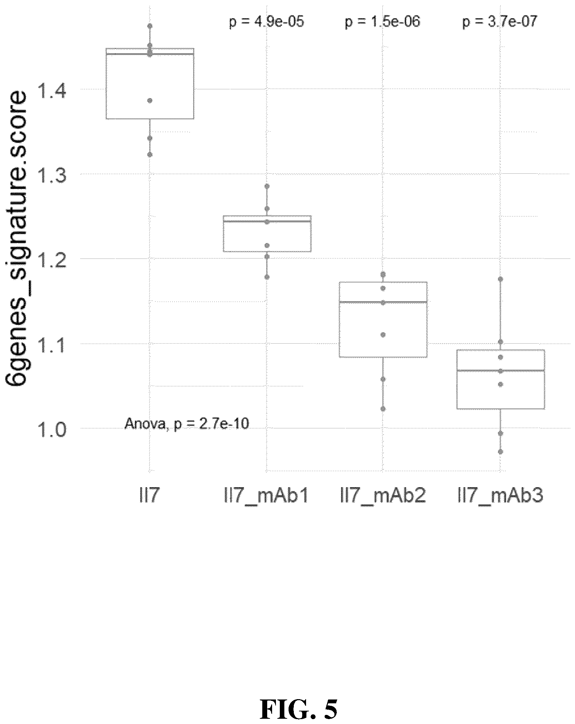

**Figure 5****:** Boxplot representation of the 6 genes ssGSEA signature with *BCL2, CISH, PTGER2, DPP4, SOCS2* and *FLT3LG* gene expression calculated with GSVA R package on RNAseq sequencing of human PBMCs preincubated with 3 anti-human IL7Ra mAb (mAb1 : N13B2-hVL6 (disclosed in WO2018/104483), mAb2 : MD707-13 (anti-site 1 antibody disclosed in WO2013/056984), mAb3 : GSKWO2011 (1A11 disclosed in GSK patent WO2011/094259)) and stimulated 3h with recombinant IL7. A significant and high decrease of the ssGSEA signature related to the IL7/IL7R pathway was observed for the 3 antibodies with these 6 genes.

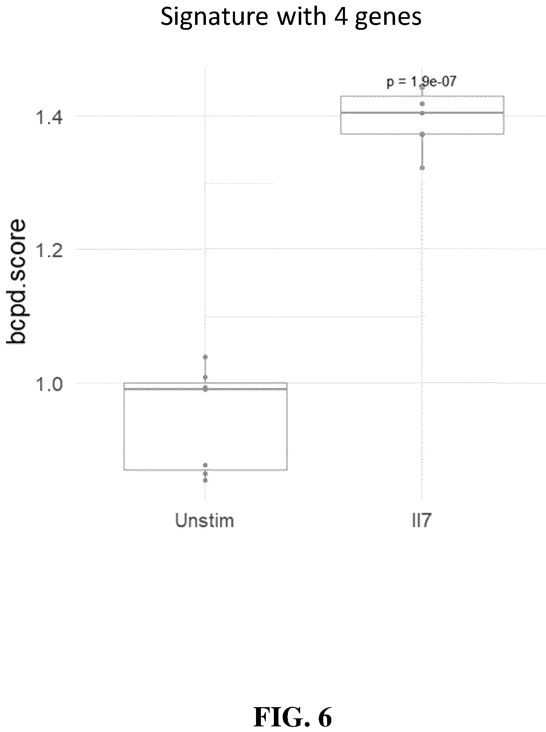

**Figure 6****:** Boxplot representation of the 4 genes ssGSEA signature with *BCL2, CISH, PTGER2, DPP4* gene expression calculated with GSVA R package on RNAseq sequencing of human PBMCs stimulated with recombinant IL7. A significant and high increase of the ssGSEA signature related to the IL7/IL7R pathway was observed.
**Figure 7****:** Boxplot representation of the 6 genes ssGSEA signature with *BCL2, CISH, PTGER2, DPP4, FLT3LG* and *SOCS2* gene expression calculated with GSVA R package on RNAseq sequencing of human PBMCs stimulated with recombinant IL7. A significant and high increase of the ssGSEA signature related to the IL7/IL7R pathway was observed.

### EXAMPLES

RNA with rRNA and globulin depletion was 150-base pair-end sequenced on an Illumina HiSeq by Genewiz company from paxgen blood samples of anti-IL7R antagonist antibody on healthy volunteers' population.

The raw files fastq were evaluated for the quality of sequencing by fastQC bioinformatic tool. Reads were pseudo-aligned to CRGh38/hg38 using bioinformatic tool salmon with the index preparation option -genecode, and raw count table and normalized TPM table for reads number for each gene were generated using bioinformatic tool salmon with the option -geneMap. Pseudo mapping quality reads were evaluated using fastQC bioinformatic tool, with an average of 20 to 30M reads with more than 90% bases ≥ 30 for all the samples.

This healthy volunteer's cohort includes 22 peripheral blood mononuclear cells (PBMC) samples before anti-human IL7R antagonist antibody (N13B2-hVL6) (10mg/kg) or placebo injection, paired with peripheral blood mononuclear cells (PBMC) samples collected at Day 15 after injection, and Day 57 after injection for 4 of them, for a total cohort of 48 samples. Only one sample did not pass the different quality controls and was removed to the study.

The inventors identified 6 genes really implicated on an IL7/IL7R signalling pathway: BCL2, CISH, SOCS2, FLT3LG, PTGER2 and DPP4.

To evaluate the efficiency of a gene signature with these genes, a single sample GSEA signature was calculated (ssGSEA).

A significant and high decrease of the 6 genes ssGSEA signature was observed during the treatment at D15 for samples from Healthy volunteers treated by N13B2-hVL6 but not for samples from Healthy volunteers treated by placebo (**Figure 1A**)**.** This decrease was observed even at D57 after the N13B2-hVL6 injection, which was consistent with the long-term detection (more than 3 months) of the antibody on the serum of the Healthy volunteers treated with N13B2-hVL6 (**Figure 1B**).

A significant and high decrease was also observed with only 4 genes ssGSEA signature during the treatment at D15 for samples from Healthy volunteers treated by N13B2-hVL6 but not for samples from Healthy volunteers treated by placebo (**Figure 2A**)**.** As with the 6 genes signature, this decrease was observed even at D57 after the N13B2-hVL6 injection (**Figure 2B**).

A higher sensitivity with 4 and 6 genes ssGSEA signatures after short- and long-term treatment is observed in comparison to the ssGSEA analysis of only one gene as shown in the Table 1 below:

**Table 1: ssGSEA analysis of the 4 and 6 genes performed on the 10 mg:kg N13B2-hVL6 treated subject samples**

| | Anova.test | t.test D1 vs D15 | **log2FC = D15** - **D1** | t.test D1 vs D57 | **log2FC = D57** - **D1** |
|---|---|---|---|---|---|
| | 10 mg/kg | | | | |
| PTGER2 | 3.6e-06 | 0.0025 | -0.24 | 0.0026 | -0.67 |
| CISH | 1.1e-07 | 5.4e-09 | -0.24 | 0.033 | -0.52 |
| BCL2 | 9.9e-07 | 2.7e-05 | -0.35 | 0.012 | -0.57 |
| DPP4 | 3.6e-06 | 0.0035 | -0.19 | 0.0093 | -0.55 |
| **PTGER2/CISH/ DPP4/BCL2** | **1.3e-08** | **5.6e-07** | **-0.26** | **0.011** | **-0.60** |
| **PTGER2/CISH/ DPP4/BCL2/ SOCS2/FLT3LG** | **8.5e-10** | **3.9e-08** | **-0.37** | **0.0027** | **-0.63** |

This new signature is strongly correlated with the biological anti-IL7R antibody antagonist effect and could be used as a tool to follow up the anti-IL7R antagonist antibody biological effect in different new trials.

The RNA seq technique is very sensitive but is an expensive technique. The gene signature was also validated by another less expensive technique, RT-qPCR, but which is less sensitive. The FLT3G and SOCS2 genes were not analyzed in RT-qPCR.

The inventors performed a RT-qPCR validation of these 4 genes as a companion test of the anti-IL7R antagonist antibody.

On 5 paired samples D1 and D15 from placebo and 5 paired samples D1 and D15 from N13B2-hVL6 treated *in vivo* volunteers (10 mg/kg) already analyzed in the RNAseq sequencing by Genewiz, a real time quantification analysis (RT-qPCR) of these 4 genes was performed.

For that, a reverse transcription (RT) with the SuperScript IV VILO master mix with EzDNASE #11766050 Thermo was performed to obtain complementary DNA (cDNA) with a Dnase treatment to eliminate genomic DNA. TaqMan^{®} Fast Advanced Master Mix #4444557(5mL) Thermo with TaqMan probes were used on a real time analyzer ViiA7 to quantify each gene and housekeeping genes (**Table 2**).

**Table 2: List of TaqMan probes used for the RT-qPCR**

| **ProductID** | **GeneID** | **Assays ID** |
|---|---|---|
| 4448892 | BCL2 | Hs01048932_g1 |
| 4448892 | CISH | Hs00367082_g1 |
| 4453320 | PTGER2 | Hs00168754_m1 |
| 4448892 | DPP4 | Hs00897405_m1 |
| 4331182 | TBP | Hs00427620_m1 |
| 4331182 | HPRT | Hs02800695_m1 |
| 4331182 | ACTB | Hs03023943_g1 |

The sum of the -DeltaCt normalized to the TBP housekeeping gene of the 4 genes detected (BCL2, CISH, PTGER2, DPP4) on RT-qPCR was calculated and used as a signature.

The box plot representation of this signature (**Figure 3**) shows that the decrease observed on D15 samples from N13B2-hVL6 treated healthy volunteers' samples on RNA sequencing is also observable on the RT-qPCR analysis.

To validate this 4 genes RT-qPCR signature, a RT-qPCR analysis of these 4 genes with TBP as a housekeeping gene was performed on the 1mg/kg, 4mg/kg and 6mg/kg N13B2-hVL6 treated subject samples (**Table 3**) and a log2 Fold Change was calculated between D15 and D1 on paired samples (log2FC).

**Table 3 :RT-qPCR analysis of the 4 genes with TBP as a housekeeping gene performed on the 1mg/kg, 4mg/kg, 6 mg/kg and 10 mg/kg N13B2-hVL6 treated subject samples, with a pvalue and a log2FC calculated between D15 and D1.**

| | t.test between D1 and D15 | | | | | | | |
|---|---|---|---|---|---|---|---|---|
| | 1mg/kg | | 4mg/kg | | 6mg/kg | | 10 mg/kg | |
| | t.test | **log2FC = D15 - D1** | t.test | **log2FC = D15** - **D1** | t.test | **log2FC = D15** - **D1** | t.test | **log2FC = D15** - **D1** |
| PTGER2 | ns | -0.48 | 0.0037 | -0.8 | 0.01 | -0.41 | ns | -0.59 |
| CISH | 0.0025 | -1.26 | 0.00049 | -1.39 | 0.0049 | -0.94 | 0.00092 | -1.21 |
| BCL2 | 0.0052 | -1.04 | 0.0029 | -1.05 | 0.021 | -0.71 | 0.0013 | -0.94 |
| DPP4 | ns | -0.41 | ns | -0.57 | ns | -0.27 | 4.6e-05 | -0.57 |
| **PTGER2/CISH/ DPP4/BCL2** | **0.0029** | **-3.18** | **0.00065** | **-4.28** | **0.0034** | **-2.65** | **0.00012** | **-3.32** |

A significant decrease of the 4 genes RT-qPCR signature was observed at all doses of N13B2-hVL6 treatment.

This signature was validated with other antibodies. For that, RNAseq sequencing of human PBMCs preincubated with 3 anti-human IL7Rα mAb (mAb1: N13B2-hVL6, mAb2: MD707-13, and mAb13: GSKWO2011) and stimulated 3h with recombinant IL7 were performed.

A significant and high decrease of the ssGSEA signature related to the IL7/IL7R pathway was observed for the 3 antibodies with the 4 and 6 genes (**Figure 4** **and** **5**).

To validate that this new signature can also be correlated with IL7R agonist effect and could be used as a tool to follow up the IL7R agonist biological effect, an RNA sequencing of PBMC treated with the IL7 cytokine was performed. Freshly isolated human PBMCs from healthy donors (obtained by the Establissement Français du Sang) were incubated with 5 ng/ml of recombinant human IL-7 (AbDSerotec) for 3 h at 37 °C. Reactions were stopped on ice and the cell pellets resuspended in RLT buffer (Qiagen) containing 1% βmercaptoethanol in Rnase/Dnase free water and stored at -80 °C.

RNA was extracted using an RNA mini extraction kit according to manufacturer's instructions (Qiagen). The quality and quantity of RNA were assessed by infrared spectrometry (Nanodrop) and Agilent bioanalyzer (Agilent RNA 6000 Pico Kit). Smart-Seq2 libraries were prepared by the Broad Technology Labs and sequenced by the Broad Genomics Platform according to the SmartSeq2 protocol with some modifications (Trombetta, J. J. et al. Curr. Protoc. Mol. Biol. 107, 4.22.1-4.22.17 (2014)). Briefly, total RNA was purified using RNA-SPRI beads, polyA+ mRNA was reverse-transcribed to cDNA, and amplified cDNA was subject to transposon-based fragmentation that used dual-indexing to barcode each fragment of each converted transcript with a combination of barcodes specific to each sample.

Sequencing was carried out as paired-end 2 × 25 bp with an additional 8 cycles for each index. Data was separated by barcode and aligned using Tophat version 2.0.10 with default settings. Transcripts were quantified by the Broad Technology Labs computational pipeline using Cuffquant version 2.2.167. Briefly, data were processed through CuffNorm if 50% of the reads aligned, and if at least 100,000 pairs were aligned per sample. Normalization used the default settings, including "geometric" normalization, and expression level information as log2-transformed FPKM values (Fragments per kilobase of transcript per million mapped fragments) were used for subsequent analyses. RNA-seq results are accessible in GEO database under the accession code GSE103643 (Belarif et al. Nature communications, (2018) 9:4483).

A single sample GSEA (ssGSEA) signature was calculated based on the genes of interest BCL2, CISH, SOCS2, FLT3LG, PTGER2 and DPP4.

As shown in **Figure 6** and **7**, a significant and high increase of the 4 and 6 gene ssGSEA signature related to the IL7/IL7R pathway was observed for the samples stimulated with IL7.

A higher sensitivity is observed with 4 and 6 gene ssGSEA signature in comparison to the ssGSEA analysis of only one gene as shown in **Table 4.**

**Table 4: ssGSEA analysis of the 4 and 6 genes performed on the PBMC samples stimulated with IL7 cytokine.**

| | t.test Unstim vs IL7 | **log2FC = IL7** - **Unstim** |
|---|---|---|
| | | |
| PTGER2 | 0.0076 | 0.14 |
| CISH | 4.e-04 | 0.29 |
| BCL2 | 8.0e-08 | 0.52 |
| DPP4 | 0.0018 | 0.25 |
| **PTGER2/CISH/DPP4/BCL2** | **1.9e-07** | **0.45** |
| **PTGER2/CISH/DPP4/BCL2/SOCS2/FLT3LG** | **5.6e-09** | **0.48** |

### Conclusion

This study allowed to discover that gene expression profile of 6 genes, in particular 4 genes reflect the IL7/IL7R pathway activity. Gene expression profile of at least one of these 4 genes that can be easily quantified by RT-qPCR could be used for assessing the IL7R antagonist or agonist activity and can serve as a companion test to monitor the biological effect of an IL7R antagonist or agonist.

## Claims

1. An *in vitro* method for evaluating the therapeutic response of an IL7R antagonist or an IL7R agonist treatment in a human patient comprising the step of determining the gene expression profile of each of the following genes: *BCL2, CISH, PTGER2* and *DPP4* genes, and preferably further *FLT3LG* or *SOCS2* genes, more preferably *BCL2, CISH, PTGER2, DPP4, FLT3LG* and *SOCS2* genes in a sample of patient having received at least one dose of IL7R antagonist or IL7R agonist, wherein a lower or higher gene expression profile of said genes in said patient sample as compared to a control value is indicative that the patient is likely responsive to the IL7R antagonist or IL7R agonist treatment respectively.

2. The method according to claim 1 wherein said sample is previously collected from a patient at least 15 days after administration of at least one dose of said IL7R antagonist or IL7R agonist in said patient.

3. An *in vitro* method for assessing the likelihood of a therapeutic response in a human patient to an IL7R antagonist or agonist prior to said treatment comprising the steps of:
a) culturing a sample previously collected from a patient prior to said treatment, in presence of said IL7R antagonist or IL7R agonist,
b) determining the gene expression profile of each of the following genes: *BCL2, CISH, PTGER2* and *DPP4* genes, and preferably further *FLT3LG* or *SOCS2* genes, more preferably *BCL2, CISH, PTGER2, DPP4, FLT3LG* and *SOCS2* genes in said cultured sample, wherein a lower or higher gene expression profile of said genes in cultured sample as compared to a control value is indicative that the patient is likely responsive to the treatment with said IL7R antagonist or IL7R agonist respectively.

4. The method according to any one of claims 1 to 3 wherein said IL7R antagonist is an anti-IL7R antibody or antigen-binding fragment thereof, preferably which comprises:
a) a variable heavy chain comprising three CDRs wherein:
- VHCDR1 comprises or consists of the amino acid sequence of SEQ ID NO: 1,
- VHCDR2 comprises or consists of the amino acid sequence of SEQ ID NO: 2, and
- VHCDR3 comprises or consists of the amino acid sequence of SEQ ID NO: 3 or 11, preferably SEQ ID NO: 3 and;
b) a variable light chain comprising three CDRs wherein:
- VLCDR1 comprises or consists of the amino acid sequence of SEQ ID NO: 4 or 12, preferably SEQ ID NO: 4,
- VLCDR2 comprises or consists of the amino acid sequence of SEQ ID NO: 5 or 13, preferably SEQ ID NO: 5 and
- VLCDR3 comprises or consists of the amino acid sequence of SEQ ID NO: 6, more preferably wherein said anti-IL7R antagonist antibody or antigen-binding fragment thereof comprises a heavy chain variable domain comprising or consisting of the amino acid sequence SEQ ID NO: 7 and a light chain variable domain comprising or consisting of the amino acid sequence SEQ ID NO: 8, again more preferably said antibody or antigen-binding fragment thereof comprises a full heavy chain comprising or consisting of SEQ ID NO: 9 and a full light chain comprising or consisting of SEQ ID NO: 10.

5. The method for evaluating the therapeutic response of an IL7R antagonist according to any one of claims 1, 2 or 4 or the method for assessing the likelihood of a therapeutic response in a human patient to an IL7R antagonist according to any one of claim 3 or 4 wherein when said patient suffers from a disease associated with an increase of IL7R signaling pathway induced by IL7, preferably selected from the group consisting of: an autoimmune disease, an inflammatory disease, an allergic disease, a cancer disease, an infectious disease, a respiratory disease, and a disease related to transplantation, involving the activation or proliferation of CD127 positive diseased cells, in particular an autoimmune disease or an inflammatory disease, involving the activation or proliferation of CD127 positive cells, more preferably a chronic inflammatory disease such as Sjogren's syndrome or inflammatory bowel disease such as ulcerative colitis.

6. The method for evaluating the therapeutic response of an IL7R agonist according to any one of claims 1, 2 or 4 or the method for assessing the likelihood of a therapeutic response in a human patient to an IL7R agonist according to any one of claim 3 or 4 wherein said patient suffers from a disease associated with impaired healthy T-cell activity, preferably a cancer disease or an infectious disease which does not itself depend on the IL7R pathway for its development.

7. The method according to any one of claims 1 to 6 wherein said patient sample is a tissue sample, preferably tumor tissue containing infiltrated immune cells from a cancer patient or a blood patient sample, preferably human isolated peripheral blood mononuclear cells.

8. The method according to any one of claims 1 to 7 wherein gene expression profile is determined by detecting the mRNA expression of said genes, preferably by RT-qPCR.

9. An IL7R antagonist for use in the treatment of disease associated with the IL7R signaling pathway induced by IL7 in a human patient in need thereof, preferably selected from the group consisting of: an autoimmune disease, an inflammatory disease, an allergic disease, a cancer disease, an infectious disease, a respiratory disease, and a disease related to transplantation, involving the activation or proliferation of CD127 positive diseased cells, in particular an autoimmune disease or an inflammatory disease, involving the activation or proliferation of CD127 positive cells, more preferably a chronic inflammatory disease such as Sjogren's syndrome or inflammatory bowel disease such as ulcerative colitis, wherein said IL7R antagonist is administered to said patient after the patient has been administered with at least one dose of said IL7R antagonist and when said patient is evaluated as likely responsive to said treatment in a method as defined in any one of claims 1 to 2 and 4 to 8.

10. An IL7R antagonist for use in the treatment of disease associated with the IL7R signaling pathway induced by IL7 in a human patient in need thereof, preferably selected from the group consisting of: an autoimmune disease, an inflammatory disease, an allergic disease, a cancer disease, an infectious disease, a respiratory disease, and a disease related to transplantation, involving the activation or proliferation of CD127 positive diseased cells, in particular an autoimmune disease or an inflammatory disease, involving the activation or proliferation of CD127 positive cells, more preferably a chronic inflammatory disease such as Sjogren's syndrome or inflammatory bowel disease such as ulcerative colitis, wherein said patient is previously assessed to be likely responsive to said IL7R antagonist treatment in a method as defined in any one of claims 3 to 8.

11. The IL7R antagonist according to claim 9 or 10 for use of claim 9 or 10 wherein said IL7R antagonist is an anti-IL7R antagonist antibody or antigen-binding fragment thereof comprising:
a) a variable heavy chain comprising three CDRs wherein:
- VHCDR1 comprises or consists of the amino acid sequence of SEQ ID NO: 1,
- VHCDR2 comprises or consists of the amino acid sequence of SEQ ID NO: 2, and
- VHCDR3 comprises or consists of the amino acid sequence of SEQ ID NO: 3 or 11, preferably SEQ ID NO: 3 and;
b) a variable light chain comprising three CDRs wherein:
- VLCDR1 comprises or consists of the amino acid sequence of SEQ ID NO: 4 or 12, preferably SEQ ID NO: 4,
- VLCDR2 comprises or consists of the amino acid sequence of SEQ ID NO: 5 or 13, preferably SEQ ID NO: 5 and
VLCDR3 comprises or consists of the amino acid sequence of SEQ ID NO: 6, preferably wherein said antibody or antigen-binding fragment thereof comprises a heavy chain variable domain comprising or consisting of amino acid sequence SEQ ID NO: 7 and a light chain variable domain comprising or consisting of the amino acid sequence SEQ ID NO: 8, again more preferably wherein said antibody or antigen-binding fragment thereof comprises a full heavy chain comprising or consisting of SEQ ID NO: 9 and a full light chain comprising or consisting of SEQ ID NO: 10.

12. The IL7R agonist for use in the treatment in a human patient in need thereof of a disease associated with impaired healthy T-cell activity, preferably a cancer disease or an infectious disease which does not itself depend on the IL7R pathway for its development, wherein said IL7R agonist is administered to said patient after the patient has been administered with at least one dose of said IL7R agonist and when said patient is evaluated as likely responsive to said treatment in a method as defined in any one of claims 1 to 2 and 4 to 8.

13. The IL7R agonist for use in the treatment in a human patient in need thereof of a disease associated with impaired healthy T-cell activity, preferably a cancer disease or an infectious disease which does not itself depend on the IL7R pathway for its development, wherein said patient is previously assessed to be likely responsive to said IL7R antagonist treatment in a method as defined in any one of claims 3 to 8.

14. An *in vitro* method for selecting a compound likely effective in the treatment of disease associated with the IL7R signaling pathway induced by IL7 or a disease associated with impaired healthy T-cell activity comprising the steps of:
a) culturing a human cell, preferably an immune cell, more preferably peripheral blood mononuclear cell in presence of said compound,
b) determining the gene expression profile of each of the following genes: *BCL2, CISH, PTGER2* and *DPP4* genes, and preferably further *FLT3LG* or *SOCS2* genes, more preferably *BCL2, CISH, PTGER2, DPP4, SOCS2* and *FLT3LG* genes in said cell, and
c) selecting a compound that induces a lower or higher gene expression profile of said genes in said cell as compared to a control value,
preferably wherein gene expression profile is determined by detecting the mRNA expression of said genes, preferably by RT-qPCR.

15. A kit for use in a method according to any one of claims 1 to 14 consisting of a set of reagents that specifically detects the gene expression profile of each of the following genes:
- *BCL2, CISH, PTGER2* and *DPP4* genes,
- *BCL2, CISH, PTGER2, DPP4* and *FLT3LG* genes,
- *BCL2, CISH, PTGER2, DPP4* and *SOCS2* genes, or
- *BCL2, CISH, PTGER2, DPP4, SOCS2* and *FLT3LG* genes,
preferably wherein said reagents are primer pairs and/or probes specific of each gene.

## Patentansprüche

1. *In-vitro*-Verfahren zum Evaluieren der therapeutischen Reaktion einer IL7R-Antagonisten- oder IL7R-Agonisten-Behandlung in einem humanen Patienten, umfassend den Schritt des Bestimmens des Genexpressionsprofils von jedem der folgenden Gene: *BCL2-, CISH-, PTGER2-* und *DPP4*-Gene, und bevorzugt ferner *FLT3LG-* oder *SOCS2*-Gene, weiter bevorzugt *BCL2-, CISH-, PTGER2-, DPP4-, FLT3LG-* und *SOCS2*-Gene, in einer Probe eines Patienten, welcher mindestens eine Dosis von IL7R-Antagonist oder IL7R-Agonist erhalten hat, wobei ein niedrigeres oder höheres Genexpressionsprofil der Gene in der Patientenprobe im Vergleich zu einem Kontrollwert anzeigt, dass der Patient wahrscheinlich auf die jeweilige IL7R-Antagonisten- oder IL7R-Agonisten-Behandlung anspricht.

2. Verfahren nach Anspruch 1, wobei die Probe zuvor von einem Patienten mindestens 15 Tage nach Verabreichung von mindestens einer Dosis des IL7R-Antagonisten oder IL7R-Agonisten an den Patienten gesammelt wird.

3. *In-vitro*-Verfahren zum Bewerten der Wahrscheinlichkeit einer therapeutischen Reaktion in einem humanen Patienten auf einen IL7R-Antagonisten oder -Agonisten vor der Behandlung, umfassend die Schritte des:
a) Kultivierens einer Probe, welche zuvor von einem Patienten vor der Behandlung gesammelt wurde, in Gegenwart des IL7R-Antagonisten oder IL7R-Agonisten,
b) Bestimmens des Genexpressionsprofils von jedem der folgenden Gene: *BCL2-, CISH-, PTGER2-* und *DPP4*-Gene und bevorzugt ferner *FLT3LG-* oder SOCS2-Gene, weiter bevorzugt *BCL2-, CISH-, PTGER2-, DPP4-, FLT3LG-* und *SOCS2*-Gene in der kultivierten Probe, wobei ein niedrigeres oder höheres Genexpressionsprofil der Gene in der kultivierten Probe im Vergleich zu einem Kontrollwert anzeigt, dass der Patient wahrscheinlich auf die jeweilige Behandlung mit dem IL7R-Antagonisten oder IL7R-Agonisten anspricht.

4. Verfahren nach einem der Ansprüche 1 bis 3, wobei der IL7R-Antagonist ein Anti-IL7R-Antikörper oder Antigen-bindendes Fragment davon ist, welcher bevorzugt umfasst:
a) eine variable schwere Kette, umfassend drei CDRs, wobei:
- VHCDR1 die Aminosäuresequenz von SEQ ID NO: 1 umfasst oder daraus besteht,
- VHCDR2 die Aminosäuresequenz von SEQ ID NO: 2 umfasst oder daraus besteht, und
- VHCDR3 die Aminosäuresequenz von SEQ ID NO: 3 oder 11, bevorzugt SEQ ID NO: 3, umfasst oder daraus besteht und;
b) eine variable leichte Kette, umfassend drei CDRs, wobei:
- VLCDR1 die Aminosäuresequenz von SEQ ID NO: 4 oder 12, bevorzugt SEQ ID NO: 4, umfasst oder daraus besteht,
- VLCDR2 die Aminosäuresequenz von SEQ ID NO: 5 oder 13, bevorzugt SEQ ID NO: 5, umfasst oder aus dieser besteht und
- VLCDR3 die Aminosäuresequenz von SEQ ID NO: 6 umfasst oder daraus besteht, weiter bevorzugt, wobei der anti-IL7R-Antagonisten-Antikörper oder das Antigen-bindende Fragment davon eine variable Domäne der schweren Kette, umfassend oder bestehend aus der Aminosäuresequenz SEQ ID NO: 7, und eine variable Domäne der leichten Kette, umfassend oder bestehend aus der Aminosäuresequenz SEQ ID NO: 8, umfasst, noch weiter bevorzugt der Antikörper oder das Antigen-bindende Fragment davon eine vollständige schwere Kette, umfassend oder bestehend aus SEQ ID NO: 9, und eine vollständige leichte Kette, umfassend oder bestehend aus SEQ ID NO: 10, umfasst.

5. Verfahren zum Evaluieren der therapeutischen Reaktion eines IL7R-Antagonisten nach einem der Ansprüche 1, 2 oder 4 oder Verfahren zum Bewerten der Wahrscheinlichkeit einer therapeutischen Reaktion in einem humanen Patienten auf einen IL7R-Antagonisten nach einem der Ansprüche 3 oder 4, wobei, wenn der Patient an einer Krankheit leidet, welche mit einer durch IL7 induzierten Erhöhung des IL7R-Signalwegs assoziiert ist, bevorzugt ausgewählt aus der Gruppe bestehend aus: einer Autoimmunkrankheit, einer Entzündungskrankheit, einer allergischen Krankheit, einer Krebskrankheit, einer Infektionskrankheit, einer Atemwegskrankheit und einer Krankheit im Zusammenhang mit Transplantation, welche die Aktivierung oder Proliferation von CD127-positiven kranken Zellen involvieren, insbesondere einer Autoimmunkrankheit oder einer Entzündungskrankheit, welche die Aktivierung oder Proliferation von CD127-positiven Zellen involvieren, weiter bevorzugt eine chronische Entzündungskrankheit wie Sjögren-Syndrom oder entzündliche Darmkrankheit wie Colitis ulcerosa.

6. Verfahren zum Evaluieren der therapeutischen Reaktion eines IL7R-Agonisten nach einem der Ansprüche 1, 2 oder 4 oder Verfahren zum Bewerten der Wahrscheinlichkeit einer therapeutischen Reaktion in einem humanen Patienten auf einen IL7R-Agonisten nach einem der Ansprüche 3 oder 4, wobei der Patient an einer Krankheit leidet, welche mit beeinträchtigter Aktivität gesunder T-Zellen assoziiert ist, bevorzugt einer Krebskrankheit oder einer Infektionskrankheit, welche selbst nicht vom IL7R-Signalweg für deren Entwicklung abhängt.

7. Verfahren nach einem der Ansprüche 1 bis 6, wobei die Patientenprobe eine Gewebeprobe, bevorzugt Tumorgewebe, beinhaltend infiltrierte Immunzellen von einem Krebspatienten, oder eine Blutpatientenprobe, bevorzugt menschliche isolierte periphere mononukleäre Blutzellen, ist.

8. Verfahren nach einem der Ansprüche 1 bis 7, wobei das Genexpressionsprofil durch Detektieren der mRNA-Expression der Gene, bevorzugt durch RT-qPCR, bestimmt wird.

9. IL7R-Antagonist zur Verwendung bei der Behandlung von Krankheiten, welche mit dem durch IL7 induzierten IL7R-Signalweg assoziiert sind, in einem humanen Patienten, welcher dessen bedarf, bevorzugt ausgewählt aus der Gruppe bestehend aus: einer Autoimmunkrankheit, einer Entzündungskrankheit, einer allergischen Krankheit, einer Krebskrankheit, einer Infektionskrankheit, einer Atemwegskrankheit und einer Krankheit im Zusammenhang mit Transplantation, welche die Aktivierung oder Proliferation von CD127-positiven kranken Zellen involvieren, insbesondere einer Autoimmunkrankheit oder einer Entzündungskrankheit, welche die Aktivierung oder Proliferation von CD127-positiven Zellen involvieren, weiter bevorzugt eine chronische Entzündungskrankheit wie Sjögren-Syndrom oder entzündliche Darmkrankheit wie Colitis ulcerosa, wobei der IL7R-Antagonist dem Patienten verabreicht wird, nachdem dem Patienten mindestens eine Dosis des IL7R-Antagonisten verabreicht wurde und wenn der Patient als wahrscheinlich auf die Behandlung ansprechend evaluiert wird, in einem Verfahren, wie in einem der Ansprüche 1 bis 2 und 4 bis 8 definiert.

10. IL7R-Antagonist zur Verwendung bei der Behandlung von Krankheiten, welche mit dem durch IL7 induzierten IL7R-Signalweg assoziiert sind, in einem humanen Patienten, welcher dessen bedarf, bevorzugt ausgewählt aus der Gruppe bestehend aus: einer Autoimmunkrankheit, einer Entzündungskrankheit, einer allergischen Krankheit, einer Krebskrankheit, einer Infektionskrankheit, einer Atemwegskrankheit und einer Krankheit im Zusammenhang mit Transplantation, welche die Aktivierung oder Proliferation von CD127-positiven kranken Zellen involvieren, insbesondere einer Autoimmunkrankheit oder einer Entzündungskrankheit, welche die Aktivierung oder Proliferation von CD127-positiven Zellen involvieren, weiter bevorzugt eine chronische Entzündungskrankheit wie Sjögren-Syndrom oder entzündliche Darmkrankheit wie Colitis ulcerosa, wobei der Patient zuvor als wahrscheinlich auf die IL7R-Antagonisten-Behandlung ansprechend bewertet wird, in einem Verfahren, wie in einem der Ansprüche 3 bis 8 definiert.

11. IL7R-Antagonist nach Anspruch 9 oder 10 zur Verwendung nach Anspruch 9 oder 10, wobei der IL7R-Antagonist ein Anti-IL7R-Antagonisten-Antikörper oder Antigen-bindendes Fragment davon ist, umfassend:
a) eine variable schwere Kette, umfassend drei CDRs, wobei
- VHCDR1 die Aminosäuresequenz von SEQ ID NO: 1 umfasst oder daraus besteht,
- VHCDR2 die Aminosäuresequenz von SEQ ID NO: 2 umfasst oder daraus besteht, und,
- VHCDR3 die Aminosäuresequenz von SEQ ID NO: 3 oder 11, bevorzugt SEQ ID NO: 3, umfasst oder daraus besteht;
b) eine variable leichte Kette, umfassend drei CDRs, wobei:
- VLCDR1 die Aminosäuresequenz von SEQ ID NO: 4 oder 12, bevorzugt SEQ ID NO: 4, umfasst oder daraus besteht,
- VLCDR2 die Aminosäuresequenz von SEQ ID NO: 5 oder 13, bevorzugt SEQ ID NO: 5, umfasst oder daraus besteht und
VLCDR3 die Aminosäuresequenz von SEQ ID NO: 6 umfasst oder daraus besteht, wobei bevorzugt der Antikörper oder das Antigen-bindende Fragment davon eine variable Domäne der schweren Kette, umfassend oder bestehend aus Aminosäuresequenz SEQ ID NO: 7, und eine variable Domäne der leichten Kette, umfassend oder bestehend aus Aminosäuresequenz SEQ ID NO: 8, umfasst, noch weiter bevorzugt, wobei der Antikörper oder das Antigen-bindende Fragment davon eine vollständige schwere Kette, umfassend oder bestehend aus SEQ ID NO: 9, und eine vollständige leichte Kette, umfassend oder bestehend aus SEQ ID NO: 10, umfasst.

12. IL7R-Agonist zur Verwendung bei der Behandlung in einem humanen Patienten, der dessen bedarf, einer Krankheit, welche mit einer beeinträchtigten Aktivität gesunder T-Zellen assoziiert ist, bevorzugt einer Krebskrankheit oder einer Infektionskrankheit, welche selbst nicht von dem IL7R-Signalweg für ihre Entwicklung abhängt, wobei der IL7R-Agonist dem Patienten verabreicht wird, nachdem dem Patienten mindestens eine Dosis des IL7R-Agonisten verabreicht wurde und wenn der Patient als wahrscheinlich auf die Behandlung ansprechend evaluiert wird, in einem Verfahren, wie in einem der Ansprüche 1 bis 2 und 4 bis 8 definiert.

13. IL7R-Agonist zur Verwendung bei der Behandlung in einem humanen Patienten, welcher dessen bedarf, einer Krankheit, welche mit beeinträchtigter Aktivität gesunder T-Zellen assoziiert ist, bevorzugt einer Krebskrankheit oder einer Infektionskrankheit, welche selbst nicht vom IL7R-Signalweg für deren Entwicklung abhängt, wobei der Patient zuvor als wahrscheinlich auf die IL7R-Antagonisten-Behandlung ansprechend bewertet wird, in einem Verfahren, wie in einem der Ansprüche 3 bis 8 definiert.

14. *In-vitro-Verfahren* zum Selektieren einer Verbindung, welche wahrscheinlich bei der Behandlung einer Krankheit, welche mit dem durch IL7 induzierten IL7R-Signalweg assoziiert ist, oder einer Krankheit, welche mit beeinträchtigter Aktivität gesunder T-Zellen assoziiert ist, wirksam ist, umfassend die Schritte des:
a) Kultivierens einer menschlichen Zelle, bevorzugt einer Immunzelle, weiter bevorzugt einer peripheren mononukleären Blutzelle, in Gegenwart der Verbindung,
b) Bestimmens des Genexpressionsprofils von jedem der folgenden Gene: *BCL2-, CISH-, PTGER2-* und *DPP4-Gene* und bevorzugt ferner *FLT3LG-* oder *SOCS2-Gene,* weiter bevorzugt *BCL2-, CISH-, PTGER2-, DPP4-, SOCS2-* und *FLT3LG-Gene* in der Zelle, und
c) Selektieren einer Verbindung, welche ein niedrigeres oder höheres Genexpressionsprofil der Gene in der Zelle im Vergleich zu einem Kontrollwert induziert,
wobei bevorzugt das Genexpressionsprofil durch Detektieren der mRNA-Expression der Gene, bevorzugt durch RT-qPCR, bestimmt wird.

15. Kit zur Verwendung in einem Verfahren nach einem der Ansprüche 1 bis 14, bestehend aus einem Satz von Reagenzien, welcher spezifisch das Genexpressionsprofil von jedem der folgenden Gene detektiert:
- *BCL2-, CISH-, PTGER2-* und *DPP4-Gene,*
- *BCL2-, CISH-, PTGER2-, DPP4-* und *FLT3LG-Gene,*
- *BCL2-, CISH-, PTGER2-, DPP4-* und *SOCS2-Gene,* oder
- *BCL2-, CISH-, PTGER2-, DPP4-, SOCS2-* und *FLT3LG-Gene,*
wobei bevorzugt die Reagenzien Primerpaare und/oder Sonden sind, welche für jedes Gen spezifisch sind.

## Revendications

1. Procédé *in vitro* d'évaluation de la réponse thérapeutique d'un traitement par antagoniste d'IL7R ou par agoniste d'IL7R chez un patient humain comprenant l'étape de détermination du profil d'expression génique de chacun des gènes suivants : gènes *BCL2, CISH, PTGER2* et *DPP4,* et de préférence en outre gènes *FLT3LG* ou *SOCS2,* plus préférablement gènes *BCL2, CISH, PTGER2, DPP4, FLT3LG* et *SOCS2* dans un échantillon de patient ayant reçu au moins une dose d'antagoniste d'IL7R ou d'agoniste d'IL7R, dans lequel un profil d'expression génique plus faible ou plus élevé desdits gènes dans ledit échantillon de patient par comparaison à une valeur témoin indique que le patient est susceptible de répondre au traitement par antagoniste d'IL7R ou par agoniste d'IL7R, respectivement.

2. Procédé selon la revendication 1, dans lequel ledit échantillon est précédemment collecté auprès d'un patient au moins 15 jours après administration d'au moins une dose dudit antagoniste d'IL7R ou agoniste d'IL7R audit patient.

3. Procédé *in vitro* d'évaluation de la probabilité d'une réponse thérapeutique chez un patient humain à un antagoniste ou agoniste d'IL7R avant ledit traitement comprenant les étapes suivantes :
a) la mise en culture d'un échantillon précédemment collecté auprès d'un patient avant ledit traitement, en présence dudit antagoniste d'IL7R ou agoniste d'IL7R,
b) la détermination du profil d'expression génique de chacun des gènes suivants : gènes *BCL2, CISH, PTGER2* et *DPP4,* et de préférence en outre gènes *FLT3LG* ou *SOCS2,* plus préférablement gènes *BCL2, CISH, PTGER2, DPP4, FLT3LG* et *SOCS2* dans ledit échantillon en culture, dans lequel un profil d'expression génique plus faible ou plus élevé desdits gènes dans un échantillon en culture par comparaison à une valeur témoin indique que le patient est susceptible de répondre au traitement avec ledit antagoniste d'IL7R ou agoniste d'IL7R, respectivement.

4. Procédé selon l'une quelconque des revendications 1 à 3 dans lequel ledit antagoniste d'IL7R est un anticorps anti-IL7R ou un fragment de liaison à l'antigène de celui-ci, de préférence qui comprend :
a) une chaîne lourde variable comprenant trois CDR dans lequel :
- VHCDR1 comprend ou consiste en la séquence d'acides aminés de SEQ ID NO : 1,
- VHCDR2 comprend ou consiste en la séquence d'acides aminés de SEQ ID NO : 2, et - VHCDR3 comprend ou consiste en la séquence d'acides aminés de SEQ ID NO : 3 ou 11, de préférence SEQ ID NO : 3 et ;
b) une chaîne légère variable comprenant trois CDR dans lequel :
- VLCDR1 comprend ou consiste en la séquence d'acides aminés de SEQ ID NO : 4 ou 12, de préférence SEQ ID NO : 4,
- VLCDR2 comprend ou consiste en la séquence d'acides aminés de SEQ ID NO : 5 ou 13, de préférence SEQ ID NO : 5 et
- VLCDR3 comprend ou consiste en la séquence d'acides aminés de SEQ ID NO : 6, plus préférablement dans lequel ledit anticorps antagoniste anti-IL7R ou un fragment de liaison à l'antigène de celui-ci comprend un domaine variable de chaîne lourde comprenant ou consistant en la séquence d'acides aminés SEQ ID NO : 7 et un domaine variable de chaîne légère comprenant ou consistant en la séquence d'acides aminés SEQ ID NO : 8, à nouveau plus préférablement, ledit anticorps ou fragment de liaison à l'antigène de celui-ci comprend une chaîne lourde entière comprenant ou consistant en la SEQ ID NO : 9 et une chaîne légère entière comprenant ou consistant en la SEQ ID NO : 10.

5. Procédé d'évaluation de la réponse thérapeutique d'un antagoniste d'IL7R selon l'une quelconque des revendications 1, 2 ou 4 ou procédé d'évaluation de la probabilité d'une réponse thérapeutique chez un patient humain à un antagoniste d'IL7R selon l'une quelconque des revendications 3 ou 4 dans lequel ledit patient souffre d'une maladie associée à une augmentation de la voie de signalisation d'IL7R induite par IL7, de préférence sélectionnée dans le groupe consistant en : une maladie auto-immune, une maladie inflammatoire, une maladie allergique, une maladie cancéreuse, une maladie infectieuse, une maladie respiratoire, et une maladie liée à une transplantation, impliquant l'activation ou la prolifération de cellules malades CD127 positives, en particulier une maladie auto-immune ou une maladie inflammatoire, impliquant l'activation ou la prolifération de cellules CD127 positives, plus préférablement une maladie inflammatoire chronique telle que le syndrome de Sjogren ou une maladie inflammatoire de l'intestin telle qu'une rectocolite hémorragique.

6. Procédé d'évaluation de la réponse thérapeutique d'un agoniste d'IL7R selon l'une quelconque des revendications 1, 2 ou 4 ou procédé d'évaluation de la probabilité d'une réponse thérapeutique chez un patient humain à un agoniste d'IL7R selon l'une quelconque des revendications 3 ou 4 dans lequel ledit patient souffre d'une maladie associée à une activité altérée de lymphocytes T sains, de préférence une maladie cancéreuse ou une maladie infectieuse qui ne dépend pas elle-même de la voie d'IL7R pour son développement.

7. Procédé selon l'une quelconque des revendications 1 à 6, dans lequel ledit échantillon de patient est un échantillon de tissu, de préférence de tissu tumoral contenant des cellules immunitaires infiltrées provenant d'un patient cancéreux, ou un échantillon sanguin de patient, de préférence des cellules mononucléaires de sang périphérique humaines isolées.

8. Procédé selon l'une quelconque des revendications 1 à 7 dans lequel un profil d'expression génique est déterminé par détection de l'expression d'ARNm desdits gènes, de préférence par RT-qPCR.

9. Antagoniste d'IL7R pour une utilisation dans le traitement d'une maladie associée à la voie de signalisation d'IL7R induite par IL7 chez un patient humain qui en a besoin, de préférence sélectionnée dans le groupe consistant en : une maladie auto-immune, une maladie inflammatoire, une maladie allergique, une maladie cancéreuse, une maladie infectieuse, une maladie respiratoire, et une maladie liée à une transplantation, impliquant l'activation ou la prolifération de cellules malades CD127 positives, en particulier une maladie auto-immune ou une maladie inflammatoire, impliquant l'activation ou la prolifération de cellules CD127 positives, plus préférablement une maladie inflammatoire chronique telle que le syndrome de Sjogren ou une maladie inflammatoire de l'intestin telle que la rectocolite hémorragique, dans lequel ledit antagoniste d'IL7R est administré audit patient après l'administration au patient d'au moins une dose dudit antagoniste d'IL7R et lorsque ledit patient est évalué comme étant susceptible de répondre audit traitement dans un procédé tel que défini selon l'une quelconque des revendications 1 à 2 et 4 à 8.

10. Antagoniste d'IL7R pour une utilisation dans le traitement d'une maladie associée à la voie de signalisation d'IL7R induite par l'IL7 chez un patient humain qui en a besoin, de préférence sélectionnée dans le groupe consistant en : une maladie auto-immune, une maladie inflammatoire, une maladie allergique, une maladie cancéreuse, une maladie infectieuse, une maladie respiratoire, et une maladie liée à une transplantation, impliquant l'activation ou la prolifération de cellules malades CD127 positives, en particulier une maladie auto-immune ou une maladie inflammatoire, impliquant l'activation ou la prolifération de cellules CD127 positives, plus préférablement, une maladie inflammatoire chronique telle que le syndrome de Sjogren ou une maladie inflammatoire de l'intestin telle que la rectocolite hémorragique, dans lequel ledit patient est précédemment évalué comme étant susceptible de répondre audit traitement par antagoniste d'IL7R dans un procédé tel que défini selon l'une quelconque des revendications 3 à 8.

11. Antagoniste d'IL7R selon la revendication 9 ou 10 pour une utilisation selon la revendication 9 ou 10, dans lequel ledit antagoniste d'IL7R est un anticorps antagoniste anti-IL7R ou un fragment de liaison à l'antigène de celui-ci comprenant :
a) une chaîne lourde variable comprenant trois CDR dans lequel :
- VHCDR1 comprend ou consiste en la séquence d'acides aminés de SEQ ID NO : 1,
- VHCDR2 comprend ou consiste en la séquence d'acides aminés de SEQ ID NO : 2, et - VHCDR3 comprend ou consiste en la séquence d'acides aminés de SEQ ID NO : 3 ou 11, de préférence SEQ ID NO : 3 et ;
b) une chaîne légère variable comprenant trois CDR dans lequel :
- VLCDR1 comprend ou consiste en la séquence d'acides aminés de SEQ ID NO : 4 ou 12, de préférence SEQ ID NO : 4,
- VLCDR2 comprend ou consiste en la séquence d'acides aminés de SEQ ID NO : 5 ou 13, de préférence SEQ ID NO : 5 et
- VLCDR3 comprend ou consiste en la séquence d'acides aminés de SEQ ID NO : 6, de préférence dans lequel ledit anticorps ou fragment de liaison à l'antigène de celui-ci comprend un domaine variable de chaîne lourde comprenant ou consistant en une séquence d'acides aminés SEQ ID NO : 7 et un domaine variable de chaîne légère comprenant ou consistant en la séquence d'acides aminés SEQ ID NO : 8, à nouveau plus préférablement, dans lequel ledit anticorps ou fragment de liaison à l'antigène de celui-ci comprend une chaîne lourde entière comprenant ou consistant en la SEQ ID NO : 9 et une chaîne légère entière comprenant ou consistant en la SEQ ID NO : 10.

12. Agoniste d'IL7R pour une utilisation dans le traitement chez un patient humain qui en a besoin d'une maladie associée à une activité altérée de lymphocytes T sains, de préférence une maladie cancéreuse ou une maladie infectieuse qui ne dépend pas elle-même de la voie d'IL7R pour son développement, dans lequel ledit agoniste d'IL7R est administré audit patient après l'administration au patient d'au moins une dose dudit agoniste d'IL7R et lorsque ledit patient est évalué comme étant susceptible de répondre audit traitement dans un procédé tel que défini selon l'une quelconque des revendications 1 à 2 et 4 à 8.

13. Agoniste d'IL7R pour une utilisation dans le traitement chez un patient humain qui en a besoin d'une maladie associée à une activité altérée de lymphocytes T sains, de préférence une maladie cancéreuse ou une maladie infectieuse qui ne dépend pas elle-même de la voie d'IL7R pour son développement, dans lequel ledit patient est précédemment évalué comme étant susceptible de répondre audit traitement par antagoniste d'IL7R dans un procédé tel que défini selon l'une quelconque des revendications 3 à 8.

14. Procédé *in vitro* de sélection d'un composé susceptible d'être efficace dans le traitement d'une maladie associée à la voie de signalisation d'IL7R induite par IL7 ou d'une maladie associée à une activité altérée de lymphocytes T sains comprenant les étapes suivantes :
a) la mise en culture d'une cellule humaine, de préférence d'une cellule immunitaire, plus préférablement d'une cellule mononucléaire de sang périphérique en présence dudit composé,
b) la détermination du profil d'expression génique de chacun des gènes suivants : gènes *BCL2, CISH, PTGER2* et *DPP4,* et de préférence en outre gènes *FLT3LG* ou *SOCS2,* plus préférablement gènes *BCL2, CISH, PTGER2, DPP4, SOCS2* et *FLT3LG* dans ladite cellule, et
c) la sélection d'un composé qui induit un profil d'expression génique inférieur ou supérieur desdits gènes dans ladite cellule par comparaison à une valeur témoin,
de préférence dans lequel le profil d'expression génique est déterminé par détection de l'expression d'ARNm desdits gènes, de préférence par RT-qPCR.

15. Kit d'utilisation dans un procédé selon l'une quelconque des revendications 1 à 14 consistant en un ensemble de réactifs qui détecte spécifiquement le profil d'expression génique de chacun des gènes suivants :
- gènes *BCL2, CISH, PTGER2* et *DPP4,*
- gènes *BCL2, CISH, PTGER2, DPP4* et *FLT3LG,*
- gènes *BCL2, CISH, PTGER2, DPP4* et *SOCS2,* ou
- gènes *BCL2, CISH, PTGER2, DPP4, SOCS2* et *FLT3LG,*
de préférence dans lequel lesdits réactifs sont des paires d'amorces et/ou des sondes spécifiques de chaque gène.
